# EUROPEAN PATENT APPLICATION

(11) **EP 4 252 754 A1**
(43) Date of publication of application: **04.10.2023**
(21) Application number: 21897073.9
(22) Date of filing: 25.11.2021
(51) Int. Cl.: A61K 31/4706, A61P 35/00, A61P 37/02

(54) **PHARMACEUTICAL COMPOSITION FOR ENHANCING CELL KILLING, AND USE THEREOF**

(30) Priority: 25.11.2020 CN 202011339237
(71) Applicant: Shanghai Juncell Therapeutics Co., Ltd., Shanghai 201800 (CN)
(72) Inventor: JIN, Huajun, Shanghai 201800 (CN); HE, Zhou, Shanghai 201800 (CN); LI, Tiantian, Shanghai 201800 (CN); SHEN, Yongchao, Shanghai 201800 (CN); CHEN, Haibin, Shanghai 201800 (CN); HUANG, Chen, Shanghai 201800 (CN)
(74) Representative: Nederlandsch Octrooibureau
(86) International application number: PCT/CN2021/133065
(87) International publication number: WO 2022/111573

(57) **Abstract**

A pharmaceutical composition, containing chloroquine or a derivative thereof and a tumor cell killing agent, and a pharmaceutically acceptable recipient. Also provided is a use of chloroquine or a derivative thereof in preparation of a medication for treating a tumor.

## Description

### Technical Field

The invention relates to the biotechnology field, in particular to a pharmaceutical composition for enhancing cell killing by increasing the HLA expression of tumor cells and use thereof.

### Background

The immune system provides protection against cancer for the body through immune surveillance. Immune surveillance is the main method for the body to eliminate cancer cells. However, this method may lead to immune editing of cancer cells and reduce their immunogenicity. Cancer cells have multiple molecular mechanisms to block immune-mediated killing by inactivating multiple cellular components. Human leukocyte antigen (HLA) is an indispensable element for the immune system to recognize and kill cancer cells. Tumor antigens must be presented in an HLA-restricted manner to be recognized by T cell receptors. The reduction or deletion of HLA molecular expression is very common in malignant tumor cells, indicating that the reduction or deletion of HLA is an important means of tumor immune evasion. Tumor immune evasion has been shown negatively impact to the clinical outcome of cancer immunotherapy; such immunotherapy includes immune checkpoint inhibitors and cell therapies that rely on activated T cells.

Impaired expression of HLA class I (HLA-I) results in inability to activate cytotoxicity-dependent immunity. The main reasons of the decrease or loss of HLA-I expression in tumor cells are known to include at least two aspects: 1) mutation or loss of HLA-I heterozygosity, and 2) degradation of HLA-I in tumor cells through lysosomes. For the decrease or loss of HLA-I expression caused by the second aspect, if the expression level can be increased again, it will be able to effectively improve the presentation of tumor antigens and enhance the killing effect of T cells on tumors. In the current clinical practice of immune cell infusion therapy, such as tumor infiltrating lymphocyte infusion, it is usually necessary for preconditioning before cell therapy by high-dose of Fludarabine + high-dose of Cyclophosphamide for lymphatic clearance, but these two drugs used therein have significant toxicity and side effects.

### Summary of the Invention

The present invention provides a pharmaceutical composition, comprising a tumor cell killing agent, a compound of Formula (I) or a pharmaceutically acceptable salt, isomer, racemate, solvate, hydrate or prodrug, and a pharmaceutical acceptable excipient. 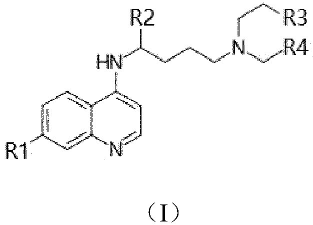 wherein, R1-R4 are each independently selected from the group consisting of H, alkyl, halogen, hydroxyl, amino, and the alkyl is optionally substituted by a group selected from the group consisting of H, C1-C6 alkyl, halogen, hydroxyl, amino, carboxyl.

In one or more embodiments, each of R1-R4 is independently selected from the group consisting of H, C1-C6 alkyl, halogen, hydroxyl, and said C1-C6 alkyl is optionally substituted by a group selected from the group consisting of H, C1-C6 alkyl, halogen, hydroxyl.

In one or more embodiments, R1 is selected from the group consisting of C1-C6 alkyl, halogen.

In one or more embodiments, R1 is selected from the group consisting of F, Cl, Br.

In one or more embodiments, R2 is selected from the group consisting of C1-C6 alkyl.

In one or more embodiments, R2 is selected from the group consisting of C1-C4 alkyl.

In one or more embodiments, R3 is selected from the group consisting of C1-C6 alkyl, halogen, hydroxyl.

In one or more embodiments, R3 is selected from the group consisting of C1-C4 alkyl, hydroxyl.

In one or more embodiments, R4 is selected from the group consisting of C1-C6 alkyl, halogen, hydroxyl.

In one or more embodiments, R4 is selected from the group consisting of C1-C4 alkyl, hydroxyl.

In one or more embodiments, R1 is Cl, R2 is selected from the group consisting of C1-C4 alkyl, R3 is selected from the group consisting of C1-C4 alkyl, hydroxyl, and R4 is selected from the group consisting of C1-C4 alkyl.

In one or more embodiments, R1 is Cl, R2 is methyl, R3 is methyl or hydroxyl, and R4 is methyl.

In one or more embodiments, the compound of Formula (I) is:

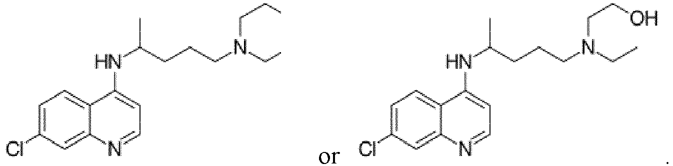

In one or more embodiments, the pharmaceutically acceptable salt is selected from the group consisting of hydrochloride, sulfate, nitrate, phosphate.

In one or more embodiments, the tumor cell killing agent is an immune cell having anti-tumor activity.

In one or more embodiments, the number of immune cells contained in the tumor cell killing reagent is at least 10⁸ cells, such as 10⁸-10¹¹ cells, preferably 10⁹-10¹¹ cells.

In one or more embodiments, the tumor cell killing agent is an immune cell having anti-tumor activity, which is in the form of a cell cryopreservant preparation.

In one or more embodiments, the cell cryopreservant preparation comprises a cryopreservant.

In one or more embodiments, the cryopreservant is a serum-free cryopreservant, preferably, is CryoStor CS10 cryopreservant from BioLifeSolutions.

In one or more embodiments, the cell density of the cell cryopreservant preparation is 1.0×10⁸ cells/mL-2.0×10⁸ cells /mL.

In one or more embodiments, the immune cells having anti-tumor activity are one or more cells selected from the group consisting of: LAK, DC, CIK, DC-CIK, CAR-T, TCR-T, NK, CAR- NK, TIL. Preferably, the immune cells having anti-tumor activity are TILs.

In one or more embodiments, the TIL is produced by a method comprising:
(1) incubating a tumor cell-containing tissue with a TIL seed cell culture medium to obtain a first population of TILs,
(2) incubating the first population of TIL with a TIL expansion medium to obtain a second population of TILs,
preferably, the method has one or more characteristics selected from the group consisting of:
a) the tumor tissue is pretreated, preferably, the pretreatment includes fragmentation and/or dissociation of the tumor tissue,
b) said tumor cell-containing tissue is a tumor tissue or body fluid of a subject having a cancer,
c) the TIL seed cell culture medium described in step (1) includes any one of the combinations of the following components: 1) IL-2, IL-6, IL-21, IFN-gamma, a TIGIT antibody, PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1beta, IL-2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15, GM-CSF, a CD137 antibody, a PD-1 antibody, TNF-alpha, serum, dual antibiotics and basal medium; 7) IL-2, IL-4, IL-10, IL-15, G-CSF, M-CSF, a CD28 antibody, an OX-40 antibody, a PD-1 antibody, serum, dual antibiotics and basal medium; 8) IL-2, IL-7, IL-15, IFN-gamma, a CD137 antibody, a CD40 antibody, an OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basic medium; 12) IL-2, IL-7, IL-15, GM-CSF, M-CSF, a PD-1 antibody, CNI-1493, serum, dual antibiotics and basic medium; 13) IL-2, IL-7, IL-15, a CD137 antibody, a CD28 antibody, a LAG3 antibody, a PD-1 antibody, Dasatinib, serum, dual antibiotics and basal medium; 14) IL-2, IL-6, IL-12, G-CSF, M-CSF, IFN-beta, IFN-gamma, a CTLA-4 antibody, a PD-1 antibody, Dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, Dasatinib, GNE-1858, serum, dual antibiotics and basal medium,
d) the incubation of step (1) lasts at least 5 days,
e) the incubation of step (1) lasts at most 20 days,
f) the incubation temperature of step (1) is 30-42°C, preferably 37°C,
g) the concentration of CO₂ in the incubation of step (1) is 5%,
h) the TIL cell expansion medium described in step (2) includes any one of the combinations of the following components: 1) 200-6000 IU/mL of IL-2, 5-100 ng/mL of IL-7, 5-100 ng/mL of IL-15, and 3-100 µg/mL of PD-1 antibodies, serum, dual antibiotics and basal medium or the proportional concentration of these components; 2) 200-6000 IU/mL of IL-2, 5-100 ng/mL of IL-7, 5-100 ng/mL of IL-15, and 1-100 µg/mL of PD-1 antibodies, serum, dual antibiotics and basal medium or the proportional concentration of these components; 3) 200-6000 IU/mL IL-2, 5-100 ng/mL IL-7, 5-100 ng/mL IL-15, 3-100 µg/mL PD-1 antibodies, 5-100 ng/mL IL-21, 5-100 ng/mL of IL-12, 1-10 µg/mL of CD3 antibodies, 1-10 µg/mL of CD28 antibodies and 200-5000 U/mL of GM-CSF, serum, dual antibiotics and basal medium or the proportional concentration of these components; 4) 200-6000 IU/mL of IL-2, 5-100 ng/mL of IL-7, 5-100 ng/mL of IL-15, 3-100 µg/mL of PD- 1 antibodies, 5-100 ng/mL IL-21, 5-100 ng/mL IL-12, 1-10 µg/mL CD3 antibodies, 0.5-10 µg/mL CD28 antibodies and 200-5000 U/mL GM-CSF, serum, dual antibiotics and basal medium or the proportional concentration of these components; 5) 200-6000 IU/mL of IL-2, 5-100 ng/mL of IL-7, 5-100 ng/mL of IL-15, 3-100 µg/mL PD-1 antibodies, 5-100 ng/mL IL-21, 5-100 ng/mL IL-12, 1-10 µg/mL CD3 antibodies, 1-10 µg/mL CD28 antibodies, 1-100 µg/mL of CD137 antibodies and 1-100 µg/mL of GITR antibodies, serum, dual antibiotics and basal medium or the proportional concentration of these components; 6) 200-6000 IU/mL of IL-2, 5-100 ng/mL IL-7, 5-100 ng/mL IL-15, 1-100 µg/mL PD-1 antibodies, 5-100 ng/mL IL-21, 5-100 ng/mL IL-12, 0.5-10 µg/mL CD3 antibodies, 1-10 µg/mL CD28 antibodies, 1-100 µg/mL CD137 antibodies and 1-100 µg/mL GITR antibodies, serum, dual antibiotics and basal medium or the proportional concentration of these components; 7) 200-6000 IU/mL IL-2, 5-100 ng/mL IL-7, 5-100 ng/mL IL-15, 3-100 µg/mL PD-1 antibodies, 1-10 µg /mL CD3 antibodies, 1-10 µg/mL CD28 antibodies, 1-100 µg/mL CD137 antibodies, 1-100 µg/mL GITR antibodies, 200-5000 U/mL GM-CSF and 1-500 µM TWS119, serum , dual antibiotics and basal medium or the proportional concentration of these components; 8) 200-6000 IU/mL IL-2, 5-100 ng/mL IL-7, 5-100 ng/mL IL-15, 1 -100 µg/mL of LAG-3 antibodies, 5-100 ng/mL of IL-21, 5-100 ng/mL of IL-12, 0.5-10 µg/mL of CD3 antibodies, 0.5-10 µg/mL of CD28 antibodies, 1- 100 µg/mL of CD137 antibodies and 1-100 µg/mL of GITR antibodies, serum, dual antibiotics and basal medium or the proportional concentration of these components;
h) the incubation of step (2) lasts at least 5 days,
i) the incubation of step (2) lasts at most 20 days,
j) the temperature of the incubation of step (2) is 30-42°C, preferably 37°C,
k) the concentration of CO₂ in the incubation of step (2) is 5%,
1) the tumor cell-containing tissue is the tumor tissue or body fluid of a subject having a cancer selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma, etc.

In one or more embodiments, the basal medium is any one selected from the group consisting of AIM-V, X-VIVO, DMEM, RPMI1640, OpTmizer^{TM}, and FUJIFII,M Irvin MHM-C.

In one or more embodiments, the serum is selected from the group consisting of human AB serum, autologous serum, or animal-derived serum. Preferably, the concentration of the serum is 1-10 v/v %,

In one or more embodiments, the TIL is produced by a method comprising:
(1) incubating a tumor cell-containing tissue with TIL seed cell culture medium to obtain a first population of TILs,
(2) contacting the first population of TILs described in (1) with any one or more of the antibodies coupled to the matrix in the aforementioned culture composition to obtain a second population of TILs,
(3) culturing the second population of TILs described in (2) in a TIL cell expansion culture medium to obtain a third population of TILs,
preferably, the method has one or more characteristics selected from the group consisting of:
a) the tumor tissue is pretreated, preferably, the pretreatment includes fragmentation and/or dissociation of the tumor tissue,
b) said tumor cell-containing tissue is a tumor tissue or body fluid of a subject having a cancer,
c) the TIL seed cell culture medium described in step (1) includes any one of the combinations of the following components: 1) IL-2, IL-6, IL-21, IFN-gamma, a TIGIT antibody, a PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1 beta, IL -2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M-CSF, a CD28 antibody, an OX-40 antibody, a PD-1 antibody, serum, dual antibiotics and basal medium; 8) IL-2, IL-7, IL-15, IFN-gamma, a CD137 antibody, a CD40 antibody, an OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basic medium; 12) IL-2, IL-7, IL-15, GM-CSF, M-CSF, a PD-1 antibody, CNI-1493, serum, dual antibiotics and basic medium; 13) IL-2, IL-7, IL-15, a CD137 antibody, a CD28 antibody, a LAG3 antibody, a PD-1 antibody, Dasatinib, serum, dual antibiotics and basal medium; 14) IL-2 , IL-6, IL-12, G-CSF, M-CSF, IFN-beta, IFN-gamma, a CTLA-4 antibody, a PD-1 antibody, Dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, Dasatinib, GNE-1858, serum, dual antibiotics and basal medium,
d) the incubation of step (1) lasts at least 5 days,
e) the incubation of step (1) lasts at most 20 days,
f) the temperature of the incubation of step (1) is 30-42°C, preferably 37°C,
g) the concentration of CO₂ in the incubation of step (1) is 5%,
h) the matrix described in step (2) is a multi-well plate, a cell culture dish or a cell culture bag,
i) the coupling described in step (2) is covalent coupling or non-covalent coupling,
j) the activating antibody coupled to the matrix in step (2) includes any one or more selected from the group consisting of a CD3 antibody, a CD28 antibody and a CD137 antibody; preferably, includes a CD3 antibody and a CD28 antibody; more preferably, includes a CD3 antibody, a CD28 antibody and a CD137 antibody,
k) the contacting in step (2) is to incubate the first population of TILs in step (1) with the antibodies coupled to the matrix; preferably, the temperature of the incubation is 30-42°C, preferably 37°C; preferably, the concentration of CO₂ of the incubation is 5%,
l) in step (2), the incubation is carried out in a medium containing the combination of components selected from the group consisting of:
   1) 200-500 IU/mL of IL-2, 5-50 ng/mL of IL-7, 5-50 ng/mL of IL-15, 1-50 µg/mL of PD-1 antibodies, 200-1000 U/mL GM-CSF, serum, dual antibiotics, and basal medium or the proportional concentration of these components; 2) 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, 1-100 µg/mL of CD137 antibodies and 1-100 µg/mL GITR antibodies, serum, dual antibiotics and basal medium or the proportional concentration of these components; 3) 200-6000 IU/mL IL-2, 5-100 ng/mL IL-7, 5-100 ng/mL IL-15, 1-100 µg/mL of PD-1 antibodies, serum, dual antibiotics and basal medium or the proportional concentration of these components; 4) 200-6000 IU/mL of IL-2, 5-100 ng/mL of IL-7, 5-100 ng/mL of IL-15, 1-100 µg/mL of PD-1 antibodies, 1-100 µg/mL of GITR antibodies and 1-500 µM of TWS119, serum, dual antibiotics and basal medium or the proportional concentration of these components; 5) 200-6000 IU/mL IL-2, 5-100 ng/mL of IL-7, 5-100 ng/mL of IL-15, 1-100 µg/mL of PD-1 antibodies, 1-100 µg/mL of TIGIT antibodies and 200-5000 U/mL of GM-CSF, serum, dual antibiotics and basal medium or the proportional concentration of these components; 6) 200-6000 IU/mL of IL-2, 5-100 ng/mL of IL-7, 5-100 ng/mL of IL-15, 1-100 µg/mL of PD-1 antibodies, 1-100 µg/mL TIGIT antibodies, 1-100 µg/mL of CD40 antibodies, 1-100 µg/mL of OX-40 antibodies and 1×10⁸-5×10⁸/mL of autologous platelets, serum, dual antibiotics and basal medium or the proportional concentration of these components; 7) 200-6000 IU/mL of IL-2, 5-100 ng/mL of IL-7, 5-100 ng/mL of IL-15, 1-100 µg/mL of PD-1 antibodies, 1-100 µg/mL of CTLA-4 antibodies, 1-100 µg/mL of CD137 antibodies, 1-100 µg/mL GITR antibodies, 200-5000 U/mL GM-CSF and 1×10⁸-5×10⁸/mLallogeneic platelets, serum, dual antibiotics and basal medium or the proportional concentration of these components,
m) the density of the first population of TILs in step (2) is 1.0×10⁵/mL~1.0× 10⁶/mL; preferably, 2.0×10⁵/mL~1.0 × 10⁶/mL; more preferably, 2.5×10⁵/mL∼3.5× 10⁶/mL.
n) the contacting in step (2) lasts for 1-3 days; preferably, lasts for 2-3 days,
o) the TIL cell expansion medium described in step (3) includes any one of the following combinations of components:
   1) 200-500 IU/mL of IL-2, 5-50 ng/mL of IL-7, 5-50 ng/mL of IL-15, 1-50 µg/mL of PD-1 antibodies, 200-1000 U/mL GM-CSF, serum, dual antibiotics, and basal medium or the proportional concentration of these components; 2) 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, 1-100 µg/mL of CD137 antibodies and 1-100 µg/mL of GITR antibodies, serum, dual antibiotics and basal medium or the proportional concentration of these components; 3) 200-6000 IU/mL of IL-2, 5-100 ng/mL of IL-7, 5-100 ng/mL of IL-15, 1-100 µg/mL of PD-1 antibodies, serum, dual antibiotics and basal medium or the proportional concentration of these components; 4) 200-6000 IU/mL of IL-2, 5-100 ng/mL of IL-7, 5-100 ng/mL of IL-15, 1-100 µg/mL of PD-1 antibodies, 1-100 µg/mL of GITR antibodies and 1-500 µM of TWS119, serum, dual antibiotics and basal medium or the proportional concentration of these components; 5) 200-6000 IU/mL of IL-2, 5-100 ng/mL of IL-7, 5-100 ng/mL of IL-15, 1-100 µg/mL of PD-1 antibodies, 1-100 µg/mL of TIGIT antibodies and 200-5000 U/mL of GM-CSF, serum, dual antibiotics and basal medium or the proportional concentration of these components; 6) 200-6000 IU/mL of IL-2, 5-100 of ng/mL IL-7, 5-100 ng/mL of IL-15, 1-100 µg/mL of PD-1 antibodies, 1-100 µg/mL of TIGIT antibodies, 1-100 µg/mL of CD40 antibodies, 1-100 µg/mL of OX-40 antibodies and 1×10⁸-5×10⁸/mL of autologous platelets, serum, dual antibiotics and basal medium or the proportional concentration of these components; 7) 200-6000 IU/mL of IL- 2, 5-100 ng/mL of IL-7, 5-100 ng/mL of IL-15, 1-100 µg/mL of PD-1 antibodies, 1-100 µg/mL of CTLA-4 antibodies, 1-100 µg/mL of CD137 antibodies, 1-100 µg/mL of GITR antibodies, 200-5000 U/mL of GM-CSF and 1×10⁸-5×10⁸/mL of allogeneic platelets, serum, dual antibiotics and basal medium or the proportional concentration of these components,
p) the incubation of step (3) lasts at least 5 days,
q) the incubation of step (3) lasts at most 20 days,
r) the temperature of the incubation of step (3) is 30-42°C, preferably 37°C,
s) the concentration of CO2 in the incubation of step (3) is 5%,
t) the tumor cell-containing tissue is a tumor tissue or body fluid of a subject having a cancer selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma, etc.

In one or more embodiments, the basal medium is any one selected from the group consisting of AIM-V, X-VIVO, DMEM, RPMI1640, OpTmizer^{TM}, and FUJIFII,M Irvin MHM-C.

In one or more embodiments, the serum is selected from the group consisting of human AB serum, the subject's own serum, or animal-derived serum. Preferably, the concentration of the serum is v/v 1-10%,

The present invention also provides the use of the compound of Formula (I) or pharmaceutically acceptable salt, isomer, racemate, solvate, hydrate or prodrug thereof in the manufacture of a drug for treating a tumor.

In one or more embodiments, the tumor is a tumor responsive to a tumor cell killing agent. Preferably, the tumor is selected from the group consisting of squamous cell carcinoma, basal cell carcinoma, adenoma, adenocarcinoma, papillary adenoma, papillary adenocarcinoma, cystadenoma, pancreatic carcinoma, cystadenocarcinoma, pleomorphic adenoma, malignant pleomorphic adenoma, papilloma, transitional epithelium cancer, fibroma, fibrosarcoma, fibrous histiocytoma, malignant fibrous histiocytoma, lipoma, liposarcoma, leiomyoma, leiomyosarcoma, rhabdomyoma, rhabdomyosarcoma, hemangioma, angiosarcoma, lymphangioma, lymphangiosarcoma, osteoma, osteosarcoma, chondroma, chondrosarcoma, synovial tumor, synovial sarcoma, lymphoma, leukemia, neurofibroma, neurofibrosarcoma, schwannoma, malignant schwannoma, glioblastoma, malignant glioma, medulloblastoma, meningioma, malignant meningioma, ganglioneuroma, neuroblastoma, pigmented nevus, melanoma, molar pregnancy, chorionic epithelium carcinoma, seminoma, dysgerminoma, embryonal carcinoma, teratoma, malignant teratoma, nasal cavity carcinoma, nasopharyngeal carcinoma, sinus carcinoma, Burkitt lymphoma, pituitary tumor, lip carcinoma, multiple myeloma /plasmacytoma, gallbladder cancer, cholangiocarcinoma, lung cancer, non-small cell lung cancer, non-Hodgkin's lymphoma, peritoneal cancer, liver cancer, cervical cancer, anal cancer, testicular cancer, myelodysplasia, bone cancer, laryngeal cancer, Hodgkin lymphoma, Waldenstrom macroglobulinemia, colorectal cancer, thyroid cancer, parathyroid cancer, mesothelioma, malignant mesothelioma, acute lymphoblastic leukemia, acute myeloid leukemia, giant lymph node hyperplasia, basal cell tumor, oral cavity cancer, oropharyngeal cancer, Kaposi's sarcoma, ovarian cancer, Langerhans cell histiocytosis, appendix cancer, dermatofibrosarcoma protuberans, chronic lymphocytic leukemia, chronic myeloid leukemia, Merkel cells cancer, brain tumor, urethral cancer, male breast cancer, bladder cancer, skin cancer, cutaneous T-cell lymphoma, prostate cancer, breast cancer, gestational trophoblastic disease, soft tissue sarcoma, ovarian germ cell tumor, renal cancer, esophageal cancer, Pheochromocytoma/paraganglioma, Sézary syndrome, fallopian tube cancer, head and neck cancer, salivary gland, Ewing sarcoma, gastric cancer, gastrointestinal carcinoid, vulvar cancer, gastrointestinal stromal tumor, small intestinal cancer, extragonadal germ cell tumor, thymoma, hypopharyngeal carcinoma, small cell lung cancer, mycosis fungoides, islet cell tumor, intraocular melanoma, vaginal carcinoma, penile carcinoma, occult primary squamous neck carcinoma, primary central nervous system lymphoma, uterine cancer, endometrial cancer, uterine sarcoma.

In one or more embodiments, the drug comprises a tumor cell killing agent.

In one or more embodiments, the tumor cell killing agent is an immune cell having anti-tumor activity.

In one or more embodiments, the number of immune cells having anti-tumor activity contained in the tumor cell killing agent is at least 10⁸, such as 10⁸-10¹¹, preferably 10⁹-10¹¹.

In one or more embodiments, the tumor cell killing agent is one or more selected from the group consisting of LAK, DC, CIK, DC-CIK, CAR-T, TCR-T, NK, CAR- NK, TIL. Preferably, the tumor cell killing agent is TIL.

In one or more embodiments, the TIL is produced by any of the aforementioned methods.

The present invention also provides a method for treating tumors, comprising administering a compound of Formula (I) or a pharmaceutically acceptable salt, isomer, racemate, solvate, hydrate or prodrug thereof and a tumor cell killing agent to a subject in need.

In one or more embodiments, the compound of Formula (I) or pharmaceutically acceptable salt, isomer, racemate, solvate, hydrate or prodrug thereof is administered simultaneously or sequentially with the tumor cell killing agent.

In one or more embodiments, the tumor cell killing agent is administered 0.5h, 1h, 6h, 12h, 18h, 1 day, 2 days, 3 days, 4 days, 5 days, 6 days, 1 week, 2 weeks, 3 weeks or 4 weeks after administration of the compound of Formula (I) or a pharmaceutically acceptable salt, isomer, racemate, solvate, hydrate or prodrug thereof.

In one or more embodiments, the tumor cell killing agent is an immune cell having anti-tumor activity.

In one or more embodiments, the number of immune cells contained in the tumor cell killing agent is at least 10⁸, such as 10⁸-10¹¹, preferably 10⁹-10¹¹.

In one or more embodiments, the tumor cell killing agent is one or more selected from the group consisting of LAK, DC, CIK, DC-CIK, CAR-T, TCR-T, NK, CAR- NK, TIL. Preferably, the tumor cell killing agent is TIL.

In one or more embodiments, the basal medium is any one selected from the group consisting of: AIM-V, X-VIVO, DMEM, RPMI1640, OpTmizer^{TM}, and FUJIFII,M Irvin MHM-C.

In one or more embodiments, the serum is selected from the group consisting of human AB serum, autologous serum, or animal-derived serum. Preferably, the concentration of the serum is 1-10 v/v %.

In one or more embodiments, the compound of Formula (I) or pharmaceutically acceptable salt, isomer, racemate, solvate, hydrate or prodrug thereof is administered via aerosol, enteral, nasal, ophthalmic, oral, parenteral, rectal, transdermal, or vaginal route.

In one or more embodiments, the tumor cell killing agent is administered via intravenous, arterial, aerosolal, enteral, nasal, ophthalmicall, buccal, parenteral, rectal, transdermal or vaginal route. Preferably, the tumor cell killing agent is administered through intravenous infusion.

The present invention also provides a method for treating solid tumors, comprising administering the aforementioned pharmaceutical composition to patients having a solid tumor.

In one or more embodiments, the pharmaceutical composition comprises autologous tumor infiltrating lymphocytes (TIL) of the patient having a solid tumor and the compound of Formula (I).

In one or more embodiments, the method includes: (1) first administering the compound of Formula (I) to the solid tumor patient; (2) infusing the autologous TILs to the solid tumor patient.

In one or more embodiments, the compound of Formula (I) is hydroxychloroquine.

In one or more embodiments, the autologous TILs are prepared by any of the methods described above.

In one or more embodiments, the hydroxychloroquine is administered at a dosage of 400 mg/day-800 mg/day; preferably at a dosage of 500 mg/day-800 mg/day; more preferably at a dosage of 600 mg/day-800 mg/day.

In one or more embodiments, the hydroxychloroquine is administered on any 1 day, 2 days or 3 days of the time period lasting from the 6th day to the 3rd day before the infusion of the autologous TILs. Preferably, the hydroxychloroquine is administered five days, four days and/or three days before the infusion of the autologous TILs.

In one or more embodiments, the method comprises: administering 400-800 mg hydroxychloroquine orally and/or administering 5-50 mg/kg cyclophosphamide by intravenous injection to the solid tumor patient 5 days before the infusion of the autologous TILs, and administering 5-50 mg/kg cyclophosphamide by intravenous injection to the solid tumor patients 4 days and the 3 days before the infusion.

In one or more embodiments, the cyclophosphamide is administered intravenously and/or orally.

In one or more embodiments, the hydroxychloroquine is administered intravenously and/or orally.

In one or more embodiments, the autologous TILs are in the form of a cell cryopreservant preparation.

In one or more embodiments, the autologous TILs are infused intravenously.

In one or more embodiments, the cell cryopreservant preparation comprises a cryopreservant.

In one or more embodiments, the infusion is performed intravenously.

In one or more embodiments, the cryopreservant is a serum-free cryopreservant, preferably CryoStor CS10 from BioLife Solutions.

In one or more embodiments, the number of infused autologous TILs is 1.0×10¹⁰-1.2×10¹¹ TILs; preferably 2.0×10¹⁰-8.0×10¹⁰ TILs; more preferably 2.0×10¹⁰-5.0×10¹⁰ TILs; still more preferably 2.0×10¹⁰-4.0×10¹⁰ TILs.

In one or more embodiments, the number of the infused autologous TILs is 1.0×10¹⁰ TILs, 1.5×10¹⁰ TILs, 2.0×10¹⁰ TILs, 2.5×10¹⁰ TILs, 3.0×10¹⁰ TILs, 3.5×10¹⁰ TILs, 4.0× 10¹⁰ TILs, 4.5× 10¹⁰ TILs, 5.0×10¹⁰ TILs, 5.5×10¹⁰ TILs, 6.0× 10¹⁰ TILs, 6.5×10¹⁰ TILs, 7.0×10¹⁰ TILs, 7.5×10¹⁰ TILs, 8.0×10¹⁰ TILs, 8.5×10¹⁰ TILs, 9.0×10¹⁰ TILs, 9.5×10¹⁰ TILs , 1.0×10¹¹ TILs, 1.2×10¹¹ TILs.

In one or more embodiments, the cell density of the cell cryopreservant preparation is 1.0×10⁸/mL-2.0×10⁸/mL.

In one or more embodiments, the autologous TILs are infused in the form of a cell cryopreservant preparation, and the infused volume is 200mL-600mL.

In one or more embodiments, the solid tumor comprises squamous cell carcinoma, basal cell carcinoma, adenoma, adenocarcinoma, papillary adenoma, papillary adenocarcinoma, cystadenoma, pancreatic carcinoma, cystadenocarcinoma, pleomorphic adenoma, malignant pleomorphic adenoma, papilloma, transitional epithelium cancer, fibroma, fibrosarcoma, fibrous histiocytoma, malignant fibrous histiocytoma, lipoma, liposarcoma, leiomyoma, leiomyosarcoma, rhabdomyoma, rhabdomyosarcoma, hemangioma, angiosarcoma, lymphangioma, lymphangiosarcoma, osteoma, osteosarcoma, chondroma, chondrosarcoma, synovial tumor, synovial sarcoma, lymphoma, leukemia, neurofibroma, neurofibrosarcoma, schwannoma, malignant schwannoma, glioblastoma, malignant glioma, medulloblastoma, meningioma, malignant meningioma, ganglioneuroma, neuroblastoma, pigmented nevus, melanoma, molar pregnancy, chorionic epithelium carcinoma, seminoma, dysgerminoma, embryonal carcinoma, teratoma, malignant teratoma, nasal cavity carcinoma, nasopharyngeal carcinoma, sinus carcinoma, Burkitt lymphoma, pituitary tumor, lip carcinoma, multiple myeloma /plasmacytoma, gallbladder cancer, cholangiocarcinoma, lung cancer, non-small cell lung cancer, non-Hodgkin's lymphoma, peritoneal cancer, liver cancer, cervical cancer, anal cancer, testicular cancer, myelodysplasia, bone cancer, laryngeal cancer, Hodgkin lymphoma, Waldenstrom macroglobulinemia, colorectal cancer, thyroid cancer, parathyroid cancer, mesothelioma, malignant mesothelioma, acute lymphoblastic leukemia, acute myeloid leukemia, giant lymph node hyperplasia, basal cell tumor, oral cavity cancer, oropharyngeal cancer, Kaposi's sarcoma, ovarian cancer, Langerhans cell histiocytosis, appendix cancer, dermatofibrosarcoma protuberans, chronic lymphocytic leukemia, chronic myeloid leukemia, Merkel cells cancer, brain tumor, urethral cancer, male breast cancer, bladder cancer, skin cancer, cutaneous T-cell lymphoma, prostate cancer, breast cancer, gestational trophoblastic disease, soft tissue sarcoma, ovarian germ cell tumor, renal cancer, esophageal cancer, Pheochromocytoma/paraganglioma, Sézary syndrome, fallopian tube cancer, head and neck cancer, salivary gland, Ewing sarcoma, gastric cancer, gastrointestinal carcinoid, vulvar cancer, gastrointestinal stromal tumor, small bowel cancer, extragonadal germ cell tumor, thymoma, hypopharyngeal carcinoma, small cell lung cancer, mycosis fungoides, islet cell tumor, intraocular melanoma, vaginal carcinoma, penile carcinoma, occult primary squamous neck carcinoma, primary central nervous system lymphoma, uterine cancer, endometrial cancer, uterine sarcoma.

In one or more embodiments, the method for treating a solid tumor may further include: evaluating efficacy.

In one or more embodiments, the efficacy evaluation includes tumor imaging evaluation and tumor marker expression level evaluation.

In one or more embodiments, said tumor imaging evaluation is performed by CT and/or MRI. Preferably, the criterion of the tumor imaging evaluation is RECIST v 1. 1.

In one or more embodiments, the expression levels of tumor markers are detected by immunohistochemistry, blood index and/or high-throughput sequencing.

Advantages of the present invention:
1) The compound of Formula (I) or pharmaceutically acceptable salt, isomer, racemate, solvate, hydrate or prodrug thereof are combined with the cell therapy of a tumor, significantly improving the efficacy of MHC restriction-dependent tumor cell therapy;
2) Compared with the high-dose fludarabine+cyclophosphamide pretreatment before TIL infusion commonly used in the field currently, the pharmaceutical composition and tumor treatment method of the present invention can completely eliminate the use of fludarabine, while significantly reducing the dosage of cyclophosphamide. Hydroxychloroquine, a drug approved for marketing years ago, has been used to treat autoimmune diseases, and produces relatively mild side effects. In addition, the dosage of cyclophosphamide is reduced, which can significantly alleviate the pain of patients during treatment and minimize the side effects caused by treatment;
3) The pharmaceutical composition of the present invention has superior clinical effects on a variety of solid tumors. And different from the common practice currently applied in the field that the subject needs high doses of IL-2 injections after TIL infusion to maintain the proliferation of TILs *in vivo,* clinically significant tumor killing and tumor suppressing effects could occur without any IL-2 injection in the subjects who received the pharmaceutical composition of the present invention post-infusion, which greatly reduces the side effects on patients. Moreover, since there is no need to inject IL-2 after administering the pharmaceutical composition of the present invention, some of the patients who were originally excluded from the treatment because of their poor physical conditions and hence unable to tolerate the side effects of IL-2 injection can also benefit from the pharmaceutical composition of the present invention. Therefore, the pharmaceutical composition of the present invention can potentially cover more subjects.

### Brief Description of the Drawings

Figure 1: *In vivo* tumor killing effects of the pharmaceutical composition derived from T008 tissue on mouse PDX model;
Figure 2: *In vivo* tumor killing effects of the pharmaceutical composition derived from T018 tissue on mouse PDX model;
Figure 3: The type I HLA changes on the surface of tumor cells in PDX model mice derived from T008 tissue after treatment with hydroxychloroquine;
Figure 4: The type I H LA changes on the surface of tumor cells in PDX model mice derived from T018 tissue after treatment with hydroxychloroquine;
Figure 5: Results of imaging evaluation of patient T009 after administration the pharmaceutical composition.

### Detailed Description

The inventors found that the expression level of type I HLA in tumor cells can be increased by administering a compound of Formula (I) or a pharmaceutically acceptable salt, isomer, racemate, solvate, hydrate or prodrug thereof (such as hydroxychloroquine (HCQ)) at the cellular level or individual level, thereby increasing the sensitization of tumor cells, improving the presentation efficiency of tumor antigens, and enhancing the killing effect of T cells on tumor cells.

The present invention first provides a pharmaceutical composition, comprising a therapeutically effective amount of the compound of Formula (I) or pharmaceutically acceptable salt, isomer, racemate, solvate, hydrate or prodrug thereof and tumor cells killing agents, and pharmaceutically acceptable recipients. The compound of Formula (I) or a pharmaceutically acceptable salt, isomer, racemate, solvate, hydrate or prodrug thereof combined with a tumor cell killing agent can obviously improve the effect of tumor cell therapy.

### Compound

Compounds of Formula (I) are shown below:

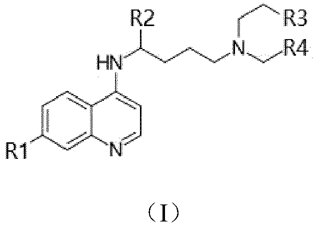

Wherein, each of R1-R4 is independently selected from the group consisting of H, C1-C6 alkyl, halogen, hydroxyl, and said C1-C6 alkyl is optionally substituted by a group selected from the group consisting of H, C1-C6 alkyl, halogen, hydroxyl.

In one or more embodiments, R1 is selected from the group consisting of C1-C6 alkyl, halogen. In one or more embodiments, R1 is selected from the group consisting of F, Cl, Br. In one or more embodiments, R2 is selected from the group consisting of C1-C6 alkyl. In one or more embodiments, R2 is selected from the group consisting of C1-C4 alkyl. In one or more embodiments, R3 is selected from the group consisting of C1-C6 alkyl, halogen, hydroxyl. In one or more embodiments, R3 is selected from the group consisting of C1-C4 alkyl, hydroxyl. In one or more embodiments, R4 is selected from the group consisting of C1-C6 alkyl, halogen, hydroxyl. In one or more embodiments, R4 is selected from the group consisting of C1-C4 alkyl, hydroxyl. In one or more embodiments, R1 is Cl, R2 is selected from the group consisting of C1-C4 alkyl, R3 is selected from the group consisting of C1-C4 alkyl, hydroxyl, and R4 is selected from the group consisting of C1-C4 alkyl.

As used herein, the term "alkyl" used alone or in combination with other terms refers to a saturated aliphatic alkyl group, including straight or branched chain alkyl groups of 1-20 carbon atoms and cyclic groups. Preferably, the alkyl group refers to a medium alkyl group containing 1-10 carbon atoms, such as methyl, ethyl, propyl, 2-isopropyl, n-butyl, isobutyl, tert-butyl, pentyl and the like. More preferably, it refers to a lower alkyl group having 1-4 carbon atoms, such as methyl, ethyl, propyl, 2-isopropyl, n-butyl, isobutyl, t-butyl and the like. Cyclic groups may be monocyclic or polycyclic, and preferably have 3-10 ring carbon atoms. Exemplary cyclic groups include cyclopropyl, cyclopropylmethyl, cyclopentyl, cyclohexyl, adamantyl, and substituted and unsubstituted bornyl, norbornyl, and norbornenyl groups. Alkyl may be substituted or unsubstituted. When substituted, the number of substituents is 1 or more, preferably 1-3, more preferably 1 or 2, and the substituents are independently selected from the group consisting of halogen, carboxyl, hydroxyl, lower alkoxy, aromatic base. Carboxy-substituted C1-C4 alkyl includes carboxy-substituted methyl, carboxy-substituted ethyl, carboxy-substituted propyl, carboxy-substituted n-butyl, carboxy-substituted isobutyl.

The term "halogen" as used herein refers to F, Cl, Br, or I. The term "haloalkyl" includes groups substituted with one or more halogen atoms, including perfluoro groups. The same is true for other groups containing the prefix "halo-". Examples of suitable haloalkyl groups are difluoromethyl, trifluoromethyl and the like.

The term "hydroxyl" refers to -OH.

The term "oxo" or the group "oxygen" refers to =O.

The term "amino" refers to -NH₂.

The term "carboxy" as used herein refers to -COOH.

As a means of simplifying the discussion and the recitation of certain terminology used throughout this application, the terms "group" and "moiety" are used to differentiate between chemical species that allow for substitution or that may be substituted and those that, in the particular embodiment of the invention, do not so allow for substitution or may not be so substituted. Thus, when the term "group" is used to describe a chemical substituent, the described chemical material includes the unsubstituted group and that group with nonperoxidic O, N, S, Si, or F atoms, for example, in the chain as well as carbonyl groups or other conventional substituents. Where the term "moiety" is used to describe a chemical compound or substituent, only an unsubstituted chemical material is intended to be included. For example, the phrase "alkyl group" is intended to include not only pure open chain saturated hydrocarbon alkyl substituents, such as methyl, ethyl, propyl, tert-butyl, and the like, but also alkyl substituents bearing further substituents known in the art, such as hydroxyl, alkoxy, alkylsulfonyl, halogen atoms, cyano, nitro, amino, carboxyl, etc. Thus, "alkyl group" includes ether groups, haloalkyls, nitroalkyls, carboxyalkyls, hydroxyalkyls, sulfoalkyls, etc. On the other hand, the phrase "alkyl moiety" is limited to the inclusion of only pure open chain saturated hydrocarbon alkyl substituents, such as methyl, ethyl, propyl, tert-butyl, and the like.

As used herein, the term "substituted" refers to a compound having a substituent comprising at least one carbon atom, nitrogen atom, oxygen atom or sulfur atom having one or more hydrogen atoms. If a substituent is described as being "substituted," it means that a non-hydrogen substituent occupies the position of a hydrogen on a carbon, nitrogen, oxygen, or sulfur. In the present invention, the alkyl, alkenyl and alkynyl groups may be substituted; for example, is a substituted or unsubstituted alkyl, a substituted or unsubstituted alkenyl, a substituted or unsubstituted alkynyl. Unless otherwise defined, a substituted group has substituent(s) at one or more appropriate positions, and when more than one position is substituted, the substituents at each substituted position may be the same or different. Substituents herein may include C1-C6 alkyl, hydroxyl, oxygen, halogen.

The term "isomer" as used herein includes: geometric isomers, enantiomers, diastereomers (such as cis-trans isomers, conformational isomers). The compounds disclosed in the present invention or their salts may contain one or more asymmetric centers, so there will be enantiomers, diastereoisomers and other stereoisomeric forms that can be defined, which can be divided, according to stereochemistry, to (R)- or (S)-, or (D)- or (L)- for amino acids. The present invention is meant to include all such possible isomers, as well as racemic and optically pure forms. Optically active (+) and (-), (R)- and (S)- or (D)- and (L)-isomers can be prepared by chiral synthons or chiral reagents, or separated by conventional techniques such as high-performance liquid chromatography with a chiral column. When the compounds described herein contain olefinic double bonds or other geometric asymmetric centers, unless otherwise stated, the compounds include both E and Z geometric isomers. Likewise, all tautomers are included.

As used herein, a "pharmaceutically acceptable" component is one that is suitable for use with humans and/or animals without undue adverse side effects (such as toxicity, irritation, and allergic response) commensurate with a reasonable benefit risk ratio. The "pharmaceutically acceptable salt" used herein includes acid salts and basic salts.

"Pharmaceutically acceptable acid salt" refers to a salt that can maintain the biological activity and properties of the free base, and such salts will not have undesired biological activity or other changes. Such salts may be formed from inorganic acids such as, but not limited to, hydrochloric, hydrobromic, sulfuric, nitric, phosphoric, and the like. Such salts may also be formed from organic acids such as, but not limited to, acetic acid, dichloroacetic acid, adipic acid, alginic acid, ascorbic acid, aspartic acid, benzenesulfonic acid, benzoic acid, 4-acetamidobenzoic acid, camphoric acid, camphorsulfonic acid, capric acid, caproic acid, caprylic acid, carbonic acid, cinnamic acid, citric acid, cyclamate, dodecylsulfonic acid, 1,2-ethanedisulfonic acid, ethanesulfonic acid, isethionic acid, formic acid, fumaric acid, galactaric acid, gentisic acid, glucoheptonic acid, gluconic acid, glucuronic acid, glutamic acid, glutaric acid, 2-oxoglutaric acid, glycerophosphate , glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, 2-naphthalenesulfonic acid, 1-naphthol-2-carboxylic acid, niacin, oleic acid, orotic acid, oxalic acid, palmitic acid, pamoic acid, propionic acid, pyroglutamic acid, pyruvic acid, water cylic acid, 4-aminosalicylic acid, sebacic acid, stearic acid, succinic acid, tartaric acid, thiocyanic acid, p-toluenesulfonic acid, trifluoroacetic acid, undecylenic acid and the like.

"Pharmaceutically acceptable basic salt" refers to a salt that can maintain the biological activity and properties of the free acid, and such salts will not have undesired biological activity or other changes. These salts are prepared by adding an inorganic or organic base to the free acid. Salts derived from inorganic bases include, but are not limited to, slats of sodium, potassium, lithium, ammonium, calcium, magnesium, iron, zinc, copper, manganese, aluminum and the like. Preferred inorganic salts are slats of ammonium, sodium, potassium, calcium and magnesium. Salts derived from organic bases include, but are not limited to, primary, secondary, and tertiary ammonium salts; substituted amines include naturally substituted amines, cyclic amines, and basic ion exchange resins, such as ammonia, isopropylamine, trimethylamine, diethylamine, triethylamine, tripropylamine, diethanolamine, ethanolamine, tannol, 2-dimethylaminoethanol, 2-diethylaminoethanol, dicyclohexylamine, lysine, arginine, histidine, caffeine, procaine, halamine, choline, betaine, phenethylbenzylamine, N,N'-bisbenzylethylenediamine, ethylenediamine, glucosamine, methylglucosamine, theobromine, three ethanolamine, tromethamine, purine, piperazine, piperidine, N-ethylpiperidine, polyamide resin, and the like. Preferred organic bases are isopropylamine, diethylamine, ethanolamine, trimethylamine, dicyclohexylamine, choline and caffeine.

Often crystallization will result in solvated products of the disclosed compounds. As used herein, the term "solvate" refers to a polymer comprising one or more molecules of a compound disclosed herein and one or more solvent molecules. The solvent may be water, in which case the solvate may be a hydrate. Alternatively, the solvent may also be an organic solvent. Therefore, the compounds disclosed in this patent can exist as hydrates, including monohydrates, dihydrates, hemihydrates, sesquihydrates, trihydrates, tetrahydrates and the like, and can also be exist as corresponding solvated products. The compounds disclosed herein may be true solvates, while in other cases, the compounds disclosed herein may retain only some water, or a mixture of water and some solvent.

The "prodrug of the compound" refers to that when taken in an appropriate way, the prodrug of the compound undergoes metabolism or chemical reaction in the patient's body and converts into a compound of Formula (I), or a salt or solution consists of a compound of chemical Formula (I).

In the present invention, the term "comprise" means that various components can be used together in the mixture or composition of the present invention. Therefore, the term "mainly consist of••••••" and "consist of••••••" is included in the term "comprise".

### Tumor Cell Killing Agents

The tumor cell killing agents described herein specifically refer to immune cells with anti-tumor activity, including but not limited to: LAK, DC, CIK, CTL, DC-CIK, DC-CTL, CAR-T, TCR-T, CAR-NK, TIL. As shown in the examples, antigen presentation efficiency and tumor cell killing effect are significantly improved in individuals who are administered with tumor cell killing agents (such as TILs) concurrently with or 0.5h, 1h, 6h, 12h, 18h, 1 day, 2 days, 3 days, 1 week, 2 weeks, 3 weeks or 4 weeks after administration of the compound of formula (I) or pharmaceutically acceptable salt, isomer, racemate, solvate, hydrate or prodrug thereof.

LAK (lymphokine activated killer cells) LAK cells are killer cells that can kill NK insensitive tumor cells after NK cells or T cells are induced by high doses of cytokines such as IL-2 in vitro. LAK is a killer cell with broad-spectrum anti-tumor effect. LAK cell adoptive immunotherapy can be combined with direct injection of cytokines such as IL-2 to treat a tumor.

DC (dendritic cell) therapy: inducing DC in vitro from the patient's autologous monocytes, preparing dendritic cells loaded with tumor antigens by loading corresponding tumor antigens, and injecting these dendritic cells into the body to stimulate the proliferation of tumor-killing lymphocytes. DC therapy can exert long-term tumor monitoring function and tumor-killing effects, therefore eradicating tumors.

CIK (cytokine-induced killer) or NK cell-like T lymphocytes are killer cells induced by a variety of cytokines, which have both the strong anti-tumor activity of T lymphocytes and the non-MHC-restricted tumor killing effects of NK cells.

CTL (cytotoxic T lymphocyte) is a CD8+ T lymphocyte, which has the ability to directly kill other cells. Through direct contacting with target cells, CTL induces apoptosis of target cells that show antigen specificity relative to CTL in an MHC-restricted manner. CTL is important in the body's tumor immunity and anti-virus infection. CTLs are considered to be a key defense mechanism in viral infections such as HIV-1. CTL is also considered to be an important component of anti-tumor immunity.

CAR-T (Chimeric Antigen Receptor T-Cell) expresses chimeric antigen receptors on the surface of T cells. A chimeric antigen receptor comprises an extracellular polypeptide that recognizes a tumor antigen, a hinge region, a transmembrane region and one or more intracellular signal regions. CAR-T activates the ITAM signal transmission of the intracellular signal CD3 zeta or FcεRIγ through the extracellular single-chain antibody fragment that specifically recognizes the tumor antigen. However, the first-generation CAR receptors lack the co-stimulatory signal of T cells, so T cells can only be effective transiently, for that the T cells exist in the body for a short time, and secrete less cytokines. The second-generation and third-generation CARs combine the two signals required for T cell activation, and directly connect the second signal (such as CD28 or/and 4-1BB intracellular signaling region) to the CD3 zeta molecule.

TCR-T (T Cell Receptor T-Cell) cell therapy is similar to CAR-T cell therapy. T cells are activated and induced to kill cancer cells by expressing a new T cell receptor (TCR) that can recognize cancer cells. TCR-T can be used in solid tumors, targeting some targets that are generally not expressed by normal cells but may be expressed by tumor cells. TCR-T recognizes the target antigen peptide presented by the MHC protein of tumor cells through a new artificial TCR. For a certain target-specific TCR, a specific MHC (HLA typing) is also required. Therefore, TCR-T is MHC restricted. However, unlike CAR-T whose targets are limited to tumor membrane proteins, TCR-T can target any "non-self" protein, including intracellular proteins. The mechanism of TCR-T is closer to the natural mechanism of T cells, and the toxicity and side effects are relatively low.

NK (natural killer) cells can directly kill tumor cells in a non-specific mean, and this natural killing activity is not limited by MHC, does not depend on antibodies, and does not require antigen sensitization. Therapeutic NK cells can be used in adoptive transfer therapy of a cancer. NK cells, unlike T cells, do not release large amounts of inflammatory proteins that may cause a cytokine storm. Another advantage of NK cell is its versatility, NK cells from healthy people or umbilical cord blood could be used, thereby reducing waiting time and cost for treatment.

Similar to CAR-T, CAR-NK uses NK cells instead of T cells, which has the high affinity and targeting of CAR and the safety and versatility of NK cells. It is reported that the objective response rate of CAR-NK cell therapy reached 73%, and did not cause similar complications of CAR-T therapy.

TIL (tumor infiltrating lymphocytes) are some infiltrating T cells during the formation of tumors. TIL is an important part of tumor cell immunotherapy. The preparation of TILs includes: isolating a small amount of TILs from the patient's tumor tissue, optionally screening the tumor-specific TILs, expanding the TILs to a certain level (for example, above 10¹⁰), and infused the TILs into the patient having the tumor.

The above-mentioned immune cells with anti-tumor activity can be used in combination, such as DC-CIK immunotherapy. Therefore, the tumor cell killing agent described herein may include one or more different above-mentioned immune cells, and different types of immune cells may have different anti-tumor activities (such as killing tumor cells of different types and/or sources), or may have same or similar anti-tumor activity (e.g. killing the same tumor cells). In addition, the same kind of immune cells can also have different antitumor activities.

### Pharmaceutical Composition

Herein, pharmaceutically acceptable excipients include but not limited to pharmaceutically acceptable diluents, carriers, solubilizers, emulsifiers, preservatives and/or adjuvants. The excipient is preferably nontoxic to recipients at the dosages and concentrations employed. In certain embodiments, pharmaceutical compositions may contain ingredients for improving, maintaining or preserving, for example, the pH, osmolarity, viscosity, clarity, color, isotonicity, odor, sterility, stability, dissolution or the release rate, absorption or penetration. These substances are known from the prior art, e.g. see REMINGTON'S PHARMACEUTICAL SCIENCES, 18th Edition, A. R. Genrmo ed., 1990, Mack Publishing Company. The optimal pharmaceutical composition may be determined by the intended route of administration, mode of delivery, and desired dosage.

Pharmaceutical compositions of the invention may be selected for parenteral delivery, for inhalation or delivery through the alimentary canal such as oral, for example for intravenous infusion delivery. The manufacture of such compositions is within the skill of the art. Other pharmaceutical compositions will also be apparent to those skilled in the art, including formulations comprising a compound of Formula (I) and a tumor cell killing agent in a sustained or controlled release delivery formulation. Techniques for formulating a variety of other sustained or controlled delivery modes, such as liposomal vehicles, bioerodible microparticles or porous beads, and depot injections, are also known to those of skill in the art. Pharmaceutical compositions for in vivo administration are usually provided in the form of sterile formulations. Sterilization can be achieved by filtration through sterile filter membranes. When the composition is lyophilized, it can be sterilized using this method before or after lyophilization and reconstitution.

Once formulated, pharmaceutical compositions are stored in sterile vials as solutions, suspensions, gels, emulsions, solids, crystals, or as dehydrated or lyophilized powders. The formulations can be stored in ready-to-use form or reconstituted prior to administration (e.g., lyophilized). The tumor cell killing agent of the pharmaceutical composition of the present invention can be separately formulated as a cell cryopreservant preparation. The cell cryopreservant preparation includes the tumor cell killing agent and a cryopreservant. The cell cryopreservant preparation is stored under low temperature conditions, such as in dry ice or in liquid nitrogen. The cryopreservant is preferably a serum-free cryopreservant. The present invention also provides kits for producing single-dose administration units. The kits of the present invention may each contain a first container with dried medicament and a second container with an aqueous formulation. In certain embodiments of the present invention, kits are provided containing single-lumen and multi-lumen pre-filled syringes (e.g., liquid syringes and lyophilized syringes).

Dosage unit formulations for oral administration, where appropriate, can be microencapsulated. Compositions can also be prepared for extended or slow release, for example by coating or embedding particulate materials in polymers, waxes, and the like.

The compounds of the invention can also be coupled with soluble polymers as targetable carriers of the medicament. Such polymers may include polyvinylpyrrolidone, pyran copolymer, polyhydroxypropylmethacrylamide phenol, polyhydroxyethylaspartamide phenol or polyethyleneoxidepolylysine (substituted with palmitoyl residues). Furthermore, the compounds of the present invention can be coupled with biodegradable polymers used for achieving controlled medicament release, such as cross-linked or amphiphilic block copolymers of polylactic acid, poly epsilon caprolactone, polyhydroxybutyric acid, polyorthoesters, polyacetal, polydihydropyrans, polycyanoacrylate and hydrogels.

In another aspect, the invention relates to oral liquid dosage forms. Oral fluids such as solution, syrups, and elixirs can be prepared in dosage unit so that a given quantity contains a predetermined amount of a compound of the present invention. Syrups can be prepared by dissolving the compound of the invention in a suitable aqueous solution; elixirs are prepared by use of a non-toxic alcoholic solvent. Suspensions can be formulated by dispersing the compound of the invention in a non-toxic vehicle. Solubilizers and emulsifiers such as ethoxylated isostearyl alcohols and polyoxyethylene sorbitol ethers, preservatives, flavoring additives such as peppermint oil or other natural sweeteners, or saccharin or other artificial sweeteners, etc. may also be added.

In another aspect, the invention relates to parenteral administration. Pharmaceutical compositions suitable for parenteral administration include aqueous and non-aqueous sterile injectable solutions which may contain antioxidants, buffers, bacteriostats and solutes which render the formulation isotonic with the blood of the intended recipient; and aqueous and non-aqueous sterile suspensions, which may contain suspending agents and thickening agents. The compositions can be presented in unit-dose or multi-dose containers, such as sealed ampoules and vials, and can be stored in a freeze-dried (lyophilized) condition requiring only the addition of a sterile liquid carrier, such as water for injection, immediately prior to use. Extemporaneous injection solutions and suspensions can be prepared from sterile powders, granules and tablets. Parenteral compositions are usually presented in containers with sterile access pores, such as intravenous solution strips or vials with a hypodermic needle pierceable stopper.

In another aspect, the invention relates to intravenous infusion administration. After the cell cryopreservant preparation of the tumor-killing agent of the aforementioned pharmaceutical composition of the present invention is resuscitated and thawed from the low temperature, the cryopreservant can be separated from the cells and/or the cells can be pretreated by culture before intravenous infusion, or, the thawed cryopreservant preparation can be directly administered intravenously without any treatment.

In a specific embodiment, TII,s are obtained from tumor samples of pancreatic cancer patients with reference to the method disclosed in CN110785486A (which is incorporated herein by reference in its entirety). Primary tumor cells such as pancreatic tumor cells are treated with the TILs alone (control group) or in combination with a compound of Formula (I) (e.g., hydroxychloroquine) (compound group). The cells of the compound group are treated with different concentrations of the compound of Formula (I) (e.g., hydroxychloroquine) for more than 12 hours (e.g., 24 hours). Different effect-to-target ratios were used to detect the killing effect of TILs on target cells in the control group and the compound group. The results showed that the cells in the compound group can be more effectively killed by TILs, and the effect is more obvious when the concentration of hydroxychloroquine is above 20 µM; the positive rate of the cells in the compound group is significantly higher than that in the control group. The above results show that the treatment of tumor cells with hydroxychloroquine can increase the expression level of HLAs by the tumor cells, and can significantly promote the killing effect of immune effector cells.

### Methods to treat cancer

The present invention also provides a method of treating a patient, especially a tumor in a patient, by administering a pharmaceutical composition according to any embodiment of the present invention. The pharmaceutical composition comprises the compound of Formula (I) described herein or pharmaceutically acceptable salt, isomer, racemate, solvate, hydrate or prodrug thereof and the tumor cell killing agent described herein. As mentioned earlier, tumor cell killing agents are immune cells with anti-tumor activity, such as LAK, DC, CIK, CTL, DC-CIK, DC-CTL, CAR-T, TCR-T, NK, CAR-NK, TIL. In order to achieve the purpose of treatment, the number of immune cells contained in the tumor cell killing agent is at least 10⁸, such as 10⁸-10¹¹, preferably 10⁹-10¹¹. The pharmaceutical composition may be conjugated or associated with one or more additional substances described herein, such as unconjugated diagnostic agents, contrast fluids, carrier lipids or nanoparticles.

As used herein, the terms "patient", "subject", "individual", and "subject" are used interchangeably herein to include any organism, preferably an animal, more preferably a mammal (e.g., rat, mouse, dog, cats, rabbits, etc.), and most preferably humans. "Treatment" refers to the administration of a therapeutic regimen described herein to a subject to achieve at least one positive therapeutic effect (e.g., reduction in cancer cell number, reduction in tumor volume, reduction in the rate of cancer cell infiltration into surrounding organs, or reduction in the rate of tumor metastasis or tumor growth). Treatment regimens that effectively treat a patient can vary depending on factors such as the patient's disease state, age, weight, and the ability of the therapy to elicit an anticancer response in the subject.

A tumor as described herein is a tumor that responds to a tumor cell killing agent. Preferably, the tumor is selected from the group consisting of squamous cell carcinoma, basal cell carcinoma, adenoma, adenocarcinoma, papillary adenoma, papillary adenocarcinoma, cystadenoma, pancreatic carcinoma, cystadenocarcinoma, pleomorphic adenoma, malignant pleomorphic adenoma, papilloma, transitional epithelium cancer, fibroma, fibrosarcoma, fibrous histiocytoma, malignant fibrous histiocytoma, lipoma, liposarcoma, leiomyoma, leiomyosarcoma, rhabdomyoma, rhabdomyosarcoma, hemangioma, angiosarcoma, lymphangioma, lymphangiosarcoma, osteoma, osteosarcoma, chondroma, chondrosarcoma, synovial tumor, synovial sarcoma, lymphoma, leukemia, neurofibroma, neurofibrosarcoma, schwannoma, malignant schwannoma, glioblastoma, malignant glioma, medulloblastoma, meningioma, malignant meningioma, ganglioneuroma, neuroblastoma, pigmented nevus, melanoma, molar pregnancy, chorionic epithelium carcinoma, seminoma, dysgerminoma, embryonal carcinoma, teratoma, malignant teratoma, nasal cavity carcinoma, nasopharyngeal carcinoma, sinus carcinoma, Burkitt lymphoma, pituitary tumor, lip carcinoma, multiple myeloma /plasmacytoma, gallbladder cancer, cholangiocarcinoma, lung cancer, non-small cell lung cancer, non-Hodgkin's lymphoma, peritoneal cancer, liver cancer, cervical cancer, anal cancer, testicular cancer, myelodysplasia, bone cancer, laryngeal cancer, Hodgkin lymphoma, Waldenstrom macroglobulinemia, colorectal cancer, thyroid cancer, parathyroid cancer, mesothelioma, malignant mesothelioma, acute lymphoblastic leukemia, acute myeloid leukemia, giant lymph node hyperplasia, basal cell tumor, oral cavity cancer, oropharyngeal cancer, Kaposi's sarcoma, ovarian cancer, Langerhans cell histiocytosis, appendix cancer, dermatofibrosarcoma protuberans, chronic lymphocytic leukemia, chronic myeloid leukemia, Merkel cells cancer, brain tumor, urethral cancer, male breast cancer, bladder cancer, skin cancer, cutaneous T-cell lymphoma, prostate cancer, breast cancer, gestational trophoblastic disease, soft tissue sarcoma, ovarian germ cell tumor, renal cancer, esophageal cancer, Pheochromocytoma/paraganglioma, Sézary syndrome, fallopian tube cancer, head and neck cancer, salivary gland, Ewing sarcoma, gastric cancer, gastrointestinal carcinoid, vulvar cancer, gastrointestinal stromal tumor, small bowel cancer, extragonadal germ cell tumor, thymoma, hypopharyngeal carcinoma, small cell lung cancer, mycosis fungoides, islet cell tumor, intraocular melanoma, vaginal carcinoma, penile carcinoma, occult primary squamous neck carcinoma, primary central nervous system lymphoma, uterine cancer, endometrial cancer, uterine sarcoma.

The compounds, therapeutic cells or pharmaceutical compositions described herein may be used in combination with other therapeutic agents. In one embodiment, the present invention provides a product as a combined preparation for simultaneous, separate or sequential use in therapy, wherein the product comprising a compound of Formula (I), a tumor cell killing agent and at least one other therapeutic agent. Products provided as combination preparations include compositions comprising a compound of Formula (I), a tumor cell killing agent and other therapeutic agent together in the same pharmaceutical composition, or compositions comprising separated forms (such as kit forms) of the compound of Formula (I), tumor cell killing agents and other therapeutic agents.

A "therapeutic agent" as used herein is an atom, molecule or compound that is useful in the treatment of cancer or other conditions. Examples of therapeutic agents include, but are not limited to, medicaments, chemotherapeutic agents, therapeutic antibodies and antibody fragments, toxins, radioisotopes, enzymes (e.g., enzymes that divide prodrugs into cytotoxic agents at the site of tumors), nucleases, hormones, immunomodulators, antisense oligonucleotides, chelating agents, boron compounds, photoactive agents and dyes.

Chemotherapeutic agents are often cytotoxic or cytostatic in nature and can include alkylating agents, antimetabolites, antineoplastic antibiotics, topoisomerase inhibitors, mitotic inhibitory hormone therapy, targeted therapeutics, and immunotherapeutic. In some embodiments, chemotherapeutic agents useful herein include, but are not limited to: 13-cis-retinoic acid, 2-chlorodeoxyadenosine, 5-azacitidine, 5-fluorouracil, 6-mercaptopurine, 6-thioguanine, actinomycin-D, doxorubicin, Aders leukocytes, alemtuzumab, alitretinoic acid, all-trans retinoic acid, alpha interferon, hexamethylmelamine, methotrexate, amifostine, anagrelide, anastrozole, cytarabine, arsenic trioxide, aniline acridine, aminocamptothecin, aminoglutethimide, asparaginase, azacytidine, bacillus Calmette-Guerin (BCG), bendamustine, bevacizumab, bexarotene, bicalutamide, bortezomib, bleomycin, busulfan, calcium tetrahydrofolate, aerophilic factor, capecitabine, canertinib, carboplatin, carmustine, cetuximab, chlorambucil, cisplatin, cladribine, cortisone, cyclophosphamide, cytarabine, darbepoetin alpha, Dasatinib, daunorubicin, decitabine, denileukin, dexamethasone, dexasone, dexrazoxane, actinomycin D, daunorubicin, amazepam, docetaxel, Adriamycin, deoxyfluridine, eniluracil, epirubicin, epoetin alpha, erlotinib, everolimus, exemestane, estramustine, etoposide, Filgrastim, fluoxymesterone, fulvestrant, flavoxetine, floxuridine, fludarabine, fluorouracil, flutamide, gefitinib, gemcitabine, ojetuzumab, goserelin, granulocyte colony stimulating factor, granulocyte-macrophage colony-stimulating factor, hexamethylmelamine, hydrocortisone hydroxyurea, icomomab, interferon-alpha, interleukin-2, interleukin-11, isotretinoin, ixabepilone, idarubicin, imatinib mesylate, ifosfamide, irinotecan, lapatinib, lenalidomide, letrozole, leucovorin, leuprolide , liposomal Ara-C, lomustine, nitrogen mustard, megestrol, melphalan, mercaptopurine, mesna, methotrexate, methylprednisolone, mitomycin C, mitotane , mitoxantrone, nelarabine, nilutamide, octreotide, oprelleukin, oxaliplatin, paclitaxel, pamidronate sodium, pemetrexed, panitumumab, PEG interferon, pegaspasin, pegfilgrastim, PEG-L-asparaginase, pentostatin, mithromycin, prednisolone, prednisone, procarbazine, raloxifene, Rituximab, romimustine, raltitrexed, sapatitabine, samustim, satraplatin, sorafenib, sunitinib, semustine, streptozotocin, tamoxifen, Tegafur, Tegafur-uracil, Temsirolimus, Temozolomide, Teniposide, Thalidomide, Thioguanine, Thiotepa, Topotecan, Toremifene, Tositumomab, trastuzumab, tretinoin, trimetrexate, alrubicin, vincristine, vinblastine, vinblastine amide, vinorelbine, vorinostat, or zoledronic acid.

In some embodiments, antibodies and functional fragments thereof that may be used as therapeutic agents include, but are not limited to, alemtuzumab, bevacizumab, cetuximab, efrecomab, gemtuzumab, irilimomab, panitumumab, rituximab, tositumomab, and trastuzumab.

In some embodiments, toxins useful as therapeutic agents include, but are not limited to, ricin, abrin, ribonuclease (RNase), deoxyribonuclease (DNase) I, staphylococcal enterotoxin-A, pokeweed antiviral proteins, gelonin, diphtheria toxin, pseudomonas exotoxin and pseudomonas endotoxin.

In some embodiments, radioisotopes useful as therapeutic agents include, but are not limited to, ³²P, ⁸⁹Sr, ⁹⁰Y, ^{99m}Te, ⁹⁹Mo, ¹³¹I, ¹⁵³Sm, ¹⁷⁷Lu, ¹⁸⁶Re, ²¹³Bi, ²²³Ra, and ²²⁵Ac.

A "therapeutically effective amount" is an amount of a compound, therapeutic cell, or pharmaceutical composition that produces a desired therapeutic effect in an individual, such as preventing or treating the targeted disorder or alleviating symptoms associated with the disorder. A precise therapeutically effective amount is that amount of the composition which produces the most effective effect in terms of therapeutic efficacy in an individual. The amount will vary depending on a variety of factors including, but not limited to, the properties of the therapeutic compound (including activity, pharmacokinetics, pharmacodynamics, and bioavailability), individual physiological conditions (including age, sex, type and stage of disease, general physical condition, response to a given dose and type of medicaments), the pharmaceutically acceptable excipient in the formulation or the nature of excipients, and administration route. Those skilled in the clinical and pharmacological arts will be able to determine a therapeutically effective amount by routine experimentation, i.e., by monitoring the individual response to the compound and adjusting dosage accordingly. In certain embodiments, the clinician can titrate the dose and alter the route of administration to obtain optimal therapeutic effect. For additional guidance see Remington: The Science and Practice of Pharmacy, 21st ed., Univ. of Sciences in Philadelphia (USIP), Lippincott Williams & Wilkins, Philadelphia, PA, 2005.

A compound or composition of the invention may be administered in one dose or according to a dosing regimen in which a number of doses are administered at different time intervals for a given period of time. For example, doses may be administered once, twice, three or four times per day. The frequency of dosing will depend on the pharmacokinetics of compound of Formula (I) or a pharmaceutically acceptable salt, racemate, solvate, hydrate or prodrug thereof and the tumor cell killing agent in the formulation. Doses may be administered until the desired therapeutic effect is achieved or to maintain the desired therapeutic effect. Clinicians typically administer the compositions until a dosage is reached that achieves the desired effect. The composition may thus be administered as a single dose, or as two or more doses over time (which may or may not contain the same amount of the desired molecule), or as a continuous infusion through an implanted device or catheter. In certain embodiments, the compound of Formula (I) or pharmaceutically acceptable salt, isomer, racemate, solvate, hydrate or prodrug thereof is administered simultaneously or sequentially with the tumor cell killing agent. Exemplarily, the tumor cell killing agent are administered 0.5h, 1h, 6h, 12h, 18h, 1 day, 2 days, 3 days, 1 week, 2 weeks, 3 weeks or 4 weeks after the compound of Formula (I) or pharmaceutically acceptable salt, isomer, racemate, solvate, hydrate or prodrug thereof is administered.

Dosage regimens suitable for a compound or composition of the invention depend on the compound's pharmacokinetic properties, such as absorption, distribution, and half-life, which can be determined by those skilled in the art. Additionally, dosing regimens suitable for the compounds of the invention, and the duration of administration of such dosing regimens, will depend on the disease or disorder being treated, the severity of the disease or disorder, the age and physical condition of the individual being treated, the patient's medical history, the nature of existing treatments, the expected therapeutic effect and other factors within the knowledge and experience of those skilled in the art. It will also be understood by those skilled in the art that appropriate dosages may be adjusted in view of an individual's response to the dosing regimen or changes in individual requirements over time. Typical daily dosages may vary depending on the particular route chosen. A typical daily dosage for oral administration to a human weighing about 70 kg will be from about 5 mg to about 500 mg of a compound of Formula (I).

The compounds of Formula (I), tumor cell killing agents or pharmaceutical compositions described herein may be administered by any suitable route of administration. The route of administration may refer to any route of administration known in the art, including but not limited to aerosol, enteral, nasal, ocular, oral, parenteral, rectal, transdermal (e.g., topical cream or ointment, patch) or the vagina. "Transdermal" administration can be accomplished with topical creams or ointments or by means of a transdermal patch. "Parenteral" refers to routes of administration generally associated with injection, including infraorbital, infusion, intraarterial, intracapsular, intracardiac, intradermal, intramuscular, intraperitoneal, intrapulmonary, intraspinal, intrasternal, thecal Intrauterine, intravenous, subarachnoid, subcapsular, subcutaneous, transmucosal or transtracheal routes.

The present invention also provides the use of the above-mentioned compound of Formula (I) or pharmaceutically acceptable salt, isomer, racemate, solvate, hydrate or prodrug thereof and/or the tumor cell killing agent described herein in the manufacture of a medicament for treating tumors. Herein, a tumor as described herein is a tumor that responds to a tumor cell killing agent. Tumor cell killing agents are immune cells with anti-tumor activity, including but not limited to: a LAK, a DC, a CIK, a CTL, a DC-CIK, a DC-CTL, a CAR-T, a TCR-T, a CAR-NK, a TIL.

In the present invention, the above-mentioned immune cells with anti-tumor activity can be prepared by any method known in the art. The immune cells with anti-tumor activity are preferably tumor infiltrating lymphocytes (TIL). The TIL can be prepared by any method known in the art. Optionally, it can be prepared by the following method 1 and method 2.

### method I:

(1) incubating a tumor cell-containing tissue with a TIL seed cell culture medium to obtain a first population of TILs,
(2) incubating the first population of TILs with a TIL cell expansion medium to obtain a second population of TILs,
preferably, the method I has one or more characteristics selected from the group consisting of:
a) the tumor tissue is pretreated, preferably, the pretreatment includes fragmentation and/or dissociation of the tumor tissue,
b) said tumor cell-containing tissue is tumor tissue or body fluid of a subject having a cancer,
c) the TIL seed cell culture medium described in step (1) includes any one of the following combinations of the components: 1) IL-2, IL-6, IL-21, IFN-gamma, a TIGIT antibody, a PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1 beta, IL -2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M-CSF, a CD28 antibody, an OX-40 antibody, a PD-1 antibody, serum, dual antibiotics and basal medium; 8) IL-2, IL-7, IL-15, IFN-gamma, a CD137 antibody, a CD40 antibody, an OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basic medium; 12) IL-2, IL-7, IL-15, GM-CSF, M-CSF, a PD-1 antibody, CNI-1493, serum, dual antibiotics and basic medium; 13) IL-2, IL-7, IL-15, a CD137 antibody, a CD28 antibody, a LAG3 antibody, a PD-1 antibody, Dasatinib, serum, dual antibiotics and basal medium; 14) IL-2 , IL-6, IL-12, G-CSF, M-CSF, IFN-beta, IFN-gamma, a CTLA-4 antibody, a PD-1 antibody, Dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, Dasatinib, GNE-1858, serum, dual antibiotics and basal medium,
d) the incubation of step (1) lasts at least 5 days,
e) the incubation of step (1) lasts at most 20 days,
f) the temperature of the incubation of step (1) is 30-42°C, preferably 37°C,
g) the concentration of CO2 in the incubation of step (1) is 5%,
h) the TIL cell expansion medium described in step (2) includes any one of the following combinations of the components: 1) 200-6000 IU/mL of IL-2, 5-100 ng/mL of IL-7, 5-100 ng/mL of IL-15, and 3-100 µg/mL of PD-1 antibodies, serum, dual antibiotics and basal medium or the proportional concentration of these components; 2) 200-6000 IU/mL of IL-2, 5-100 ng/mL of IL-7, 5-100 ng/mL of IL-15, and 1-100 µg/mL of PD-1 antibodies, serum, dual antibiotics and basal medium or the proportional concentration of these components; 3) 200-6000 IU/mL IL-2, 5-100 ng/mL IL-7, 5-100 ng/mL IL-15, 3-100 µg/mL PD-1 antibodies, 5-100 ng/mL IL-21, 5-100 ng/mL of IL-12, 1-10 µg/mL of CD3 antibodies, 1-10 µg/mL of CD28 antibodies and 200-5000 U/mL of GM-CSF, serum, dual antibiotics and basal medium or the proportional concentration of these components; 4) 200-6000 IU/mL of IL-2, 5-100 ng/mL of IL-7, 5-100 ng/mL of IL-15, 3-100 µg/mL of PD- 1 antibodies, 5-100 ng/mL IL-21, 5-100 ng/mL IL-12, 1-10 µg/mL CD3 antibodies, 0.5-10 µg/mL CD28 antibodies and 200-5000 U/mL GM-CSF , serum, dual antibiotics and basal medium or the proportional concentration of these components; 5) 200-6000 IU/mL of IL-2, 5-100 ng/mL of IL-7, 5-100 ng/mL of IL-15, 3-100 µg/mL PD-1 antibodies, 5-100 ng/mL IL-21, 5-100 ng/mL IL-12, 1-10 µg/mL CD3 antibodies, 1-10 µg/mL CD28 antibodies , 1-100 µg/mL of CD137 antibodies and 1-100 µg/mL of GITR antibodies, serum, dual antibiotics and basal medium or the proportional concentration of these components; 6) 200-6000 IU/mL of IL-2, 5-100 ng/mL IL-7, 5-100 ng/mL IL-15, 1-100 µg/mL PD-1 antibodies, 5-100 ng/mL IL-21, 5-100 ng/mL IL-12, 0.5-10 µg/mL CD3 antibodies, 1-10 µg/mL CD28 antibodies, 1-100 µg/mL CD137 antibodies and 1-100 µg/mL GITR antibodies, serum, dual antibiotics and basal medium or the proportional concentration of these components; 7) 200-6000 IU/mL IL-2, 5-100 ng/mL IL-7, 5-100 ng/mL IL-15, 3-100 µg/mL PD-1 antibodies, 1-10 µg /mL CD3 antibodies, 1-10 µg/mL CD28 antibodies, 1-100 µg/mL CD137 antibodies, 1-100 µg/mL GITR antibodies, 200-5000 U/mL GM-CSF and 1-500 µM TWS119, serum , dual antibiotics and basal medium or the proportional concentration of these components; 8) 200-6000 IU/mL IL-2, 5-100 ng/mL IL-7, 5-100 ng/mL IL-15, 1 -100 µg/mL of LAG-3 antibodies, 5-100 ng/mL of IL-21, 5-100 ng/mL of IL-12, 0.5-10 µg/mL of CD3 antibodies, 0.5-10 µg/mL of CD28 antibodies, 1-100 µg/mL of CD137 antibodies and 1-100 µg/mL of GITR antibodies, serum, dual antibiotics and basal medium or the proportional concentration of these components;
h) the incubation of step (2) lasts at least 5 days,
i) the incubation of step (2) lasts at most 20 days,
j) the temperature of the incubation of step (2) is 30-42°C, preferably 37°C,
k) the concentration of CO2 in the incubation of step (2) is 5%,
1) the tumor cell-containing tissue is a patient with the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma, etc.

### method II:

(1) incubating a tumor cell-containing tissue with a TIL seed cell culture medium to obtain a first population of TILs,
(2) contacting the first population of TILs described in (1) with any one or more of the antibodies coupled to the matrix in the aforementioned culture composition to obtain a second population of TILs,
(3) culturing the second population of TILs described in (2) in a TIL cell expansion culture medium to obtain a third population of TILs,
preferably, the method II has one or more characteristics selected from the group consisting of:
a) the tumor tissue is pretreated, preferably, the pretreatment includes fragmentation and/or dissociation of the tumor tissue,
b) said tumor cell-containing tissue is tumor tissue or body fluid of a subject having a cancer,

The TIL seed cell culture medium described in step (1) includes any one of the following combinations of the components: 1) IL-2, IL-6, IL-21, IFN-gamma, a TIGIT antibody, a PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1 beta, IL -2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M-CSF, a CD28 antibody, an OX-40 antibody, a PD-1 antibody, serum, dual antibiotics and basal medium; 8) IL-2, IL-7, IL-15, IFN-gamma, a CD137 antibody, a CD40 antibody, an OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basic medium; 12) IL-2, IL-7, IL-15, GM-CSF, M-CSF, a PD-1 antibody, CNI-1493, serum, dual antibiotics and basic medium; 13) IL-2, IL-7, IL-15, a CD137 antibody, a CD28 antibody, a LAG3 antibody, a PD-1 antibody, Dasatinib, serum, dual antibiotics and basal medium; 14) IL-2 , IL-6, IL-12, G-CSF, M-CSF, IFN-beta, IFN-gamma, a CTLA-4 antibody, a PD-1 antibody, Dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, Dasatinib, GNE-1858, serum, dual antibiotics and basal medium,
d) the incubation of step (1) lasts at least 5 days,
e) the incubation of step (1) lasts at most 20 days,
f) the temperature of the incubation of step (1) is 30-42°C, preferably 37°C,
g) the concentration of CO2 in the incubation of step (1) is 5%,
h) the matrix described in step (2) is a multi-well plate, a cell culture dish or a cell culture bag,
i) the coupling described in step (2) is covalent coupling or non-covalent coupling,
j) the activating antibody coupled to the matrix in step (2) includes any one or more of a CD3 antibody, a CD28 antibody and a CD137 antibody; preferably, includes a CD3 antibody and a CD28 antibody; more preferably, includes a CD3 antibody, a CD28 antibody and a CD137 antibody,
k) the contacting in step (2) is to incubate the first population of TILs in step (1) with the antibodies coupled to the matrix; preferably, the temperature of the incubation is 30-42°C, preferably 37°C; preferably, the concentration of CO₂ of the incubation is 5%,
1) in step (2), the incubation is carried out in a culture medium containing any one or more of the components in the aforementioned culture composition except for the antibodies coupled to the matrix,
m) the density of the first population of TILs in step (2) is 1.0×10⁵/mL~1.0× 10⁶/mL; preferably, 2.0×10⁵/mL~1.0 × 10⁶/mL; more preferably, 2.5×10⁵/mL∼3.5× 10⁶/mL.
n) the contacting in step (2) lasts for 1-3 days; preferably, lasts for 2-3 days,
o) the TIL cell expansion medium described in step (3) includes any one of the following combinations of the components:
   1) 200-500 IU/mL of IL-2, 5-50 ng/mL of IL-7, 5-50 ng/mL of IL-15, 1-50 µg/mL of PD-1 antibody, 200-1000 U/mL GM-CSF, serum, dual antibiotics, and basal medium or the proportional concentration of these components; 2) 200-6000 IU/mL of IL-2, 5-100 ng/mL of IL-7, 5-100 ng/mL of IL-15, 1-100 µg/mL of PD-1 antibody, 5-100 ng/mL of IL-21, 5-100 ng/mL of IL-12, 1-100 µg/mL of CD137 antibody and 1-100 µg/mL of GITR antibody, serum, dual antibiotics and basal medium or the proportional concentration of these components; 3) 200-6000 of IU/mL IL-2, 5-100 ng/mL of IL-7, 5-100 ng/mL of IL-15, 1-100 µg/mL of PD-1 antibody, serum, dual antibiotics and basal medium or the proportional concentration of these components; 4) 200-6000 IU/mL of IL-2, 5-100 ng/mL of IL-7, 5-100 ng/mL of IL-15, 1-100 µg/mL of PD-1 antibody, 1-100 µg/mL of GITR antibody and 1-500 µM of TWS119, serum, dual antibiotics and basal medium or the proportional concentration of these components; 5) 200-6000 IU/mL of IL-2, 5-100 of ng/mL IL-7, 5-100 ng/mL of IL-15, 1-100 µg/mL of PD-1 antibody, 1-100 µg/mL of TIGIT antibody and 200-5000 U/mL of GM-CSF, serum, dual antibiotics and basal medium or the proportional concentration of these components; 6) 200-6000 IU/mL of IL-2, 5-100 ng/mL of IL-7, 5-100 ng/mL of IL-15, 1-100 µg/mL of PD-1 antibody, 1-100 µg/mL of TIGIT antibody, 1-100 µg/mL of CD40 antibody, 1-100 µg/mL of OX-40 antibody and 1×10⁸-5×10⁸/mL of autologous platelets, serum, dual antibiotics and basal medium or the proportional concentration of these components; 7) 200-6000 IU/mL of IL-2, 5-100 ng/mL of IL-7, 5-100 ng/mL of IL-15, 1-100 µg/mL of PD-1 antibody, 1-100 µg/mL of CTLA-4 antibody, 1-100 µg/mL of CD137 antibody, 1-100 µg/mL of GITR antibody, 200-5000 U/mL GM-CSF and 1×10⁸-5× 10⁸/mL allogeneic platelets, serum, dual antibiotics and basal medium or the proportional concentration of these components,
p) the incubation of step (3) lasts at least 5 days,
q) the incubation of step (3) lasts at most 20 days,
r) the temperature of the incubation of step (3) is 30-42°C, preferably 37°C,
s) the concentration of CO₂ in the incubation of step (3) is 5%,
t) the tumor cell-containing tissue is a tumor tissue or body fluid of subject having a cancer selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma, etc.

In method I or method II, the basal medium may be any one selected from the group consisting of AIM-V, X-VIVO, DMEM, RPMI1640, OpTmizer^{TM}, and FUJIFII,M Irvin MHM-C.

In method I or method II, the serum is selected from the group consisting of human AB serum, the subject's own serum, or animal-derived serum. Preferably, the concentration of the serum is 1-10%.

The present invention also provides a treatment device, including a memory, a processor, and a computer program stored on the memory and operable on the processor, wherein, the processor implements the following steps when executing the program: 1) administering the compound of Formula (1) or pharmaceutically acceptable salt, isomer, racemate, solvate, hydrate or prodrug thereof to a patient; 2) within 0 hours to 4 weeks, administering the tumor cell killing agent to the patient.

The present invention will be illustrated by way of specific examples below. It should be understood that these examples are merely explanatory and is not intended to limit the scope of the present invention. Unless otherwise specified, the methods and materials used in the examples are conventional materials and methods in the art.

### Example

### Example 1, Preparation of culture medium

TIL seeded cell culture medium was prepared using the following components.

| | Purchasing Source | | Purchasing Source |
|---|---|---|---|
| IL-1α | R&D 200-LA-010 | OX-40 mAb | R&D MAB10543-100 |
| CD3 mAb | abcam (ab86883) | | |
| IL-1β | R&D 201-LB-025 | LAG-3 mAb | R&D MAB23193 |
| IL-2 | R&D 201-GMP-01M | TIGIT mAb | R&D MAB7898 |
| IL-4 | R&D 204-GMP-01M | CTLA-4 mAb | R&D MAB325-500 |
| IL-6 | R&D 206-GMP-01M | PD-1 mAb | R&D MAB10861 |
| IL-7 | R&D 207-GMP-01M | TNF-α | R&D 210-GMP-100 |
| IL-9 | R&D 209-ILB-050/CF | RRx-001 | MCE HY-16438 |
| IL-10 | R&D 217-IL-025/CF | Sunitinib | MCE HY-10255A |
| IL-12 | R&D 219-GMP-01M | CNI-1493 | MCE HY-15509A |
| IL-15 | R&D 247-GMP-01M | Imatinib | MCE HY-15463 |
| IL-18 | Biotechne 9124-IL-500 | CAL-101 | MCE HY-13026 |
| IL-21 | R&D 8879-GMP-01M | Dasatinib | MCE HY-10181 |
| G-CSF | R&D 214-CS-025 | LYC-55716 | MCE HY-104037 |
| GM-CSF | R&D 215-GMP-01M | GNE-1858 | MCE HY-135892 |
| M-CSF | R&D 216-GMP-500 | methylene blue | MCE HY-14536 |
| IFN-α | R&D 11100-1 | TWS119 | MCE HY-10590 |
| IFN-β | R&D 8499-IF-010 | Human AB serum(v/v%) | Sigmaaldrich H4522 |
| IFN-γ | R&D 285-GMP-01M | 100XPS dual antibiotics | Thermo Fisher15140122 |
| CD137 mAb | R&D AF838 | AIM-V | Thermo Fisher 0870112BK |
| CD28 mAb | R&D MAB342-500 | X-VIVO 15 | Lonza BE02-060F |
| CD40 mAb | R&D MAB6321-500 | CTS^{™} OpTmizer^{™} | Thermo Fisher A1048501 |

GITR antibody was synthesized by GenScript (sequences are from SEQ ID NO: 104 and SEQ ID NO: 105 in CN103951753B).

Different TIL cell culture media were prepared according to Table 1-Table 3. The basal medium can be stored at 4°C for no more than 1 month after adding serum and dual antibiotics in proportion. Before use, the medium was taken out from 4°C, placed in a 37°C water bath to pre-warm, and then other components (interleukins, colony-stimulating factors, interferon, TNF-alpha, various antibodies and other molecules) of working concentrations were added to for TIL cell culture. The components corresponding to "coating" in Table 3 (CD3 mAb, CD28 mAb, and CD137 mAb) were not present in the medium in the form of solution components; rather, the stock solution containing these components was added to the bottom or inner walls of cell culture vessels (such as multi-well plates, plates or cell culture bags) and incubated before cell culture. After coating pretreatment, the components became a matrix-immobilized and are attached to the bottom of the culture vessel. The method of coating pretreatment can be a conventional protein coating process well known in the art. For example, a specific treatment method is as follow: coating stock solutions were prepared with the components to be coated (CD3 mAb, CD28 mAb and/or CD137 mAb) and PBS, the concentration of components contained therein being 5 µg/mL each. Then the coating stock solution was added to the above-mentioned cell culture vessel, and the bottom or inner wall of the cell culture vessel was completely covered by the coating stock solution. The cell culture vessel was incubated at 4°C overnight, then the coating stock solution was discarded, and the cell culture vessel was washed with PBS 5 times for later use. The autologous platelets in Table 3 came from the patient's own blood, and the allogeneic platelets came from the blood of healthy adults, the preparation of the platelets was carried out according to the preparation method disclosed in CN105107233B, which is incorporated herein by reference in its entirety. The mass-activity conversion relationship of some cytokines used in the following media is IL-7: 1mg=1.21 × 10⁷U; IL-15: 1mg=1.14 × 10⁷U; IL-12: 1mg=1.02 × 10⁷U; IL-21 : 1mg= 1.07 ×10⁷U

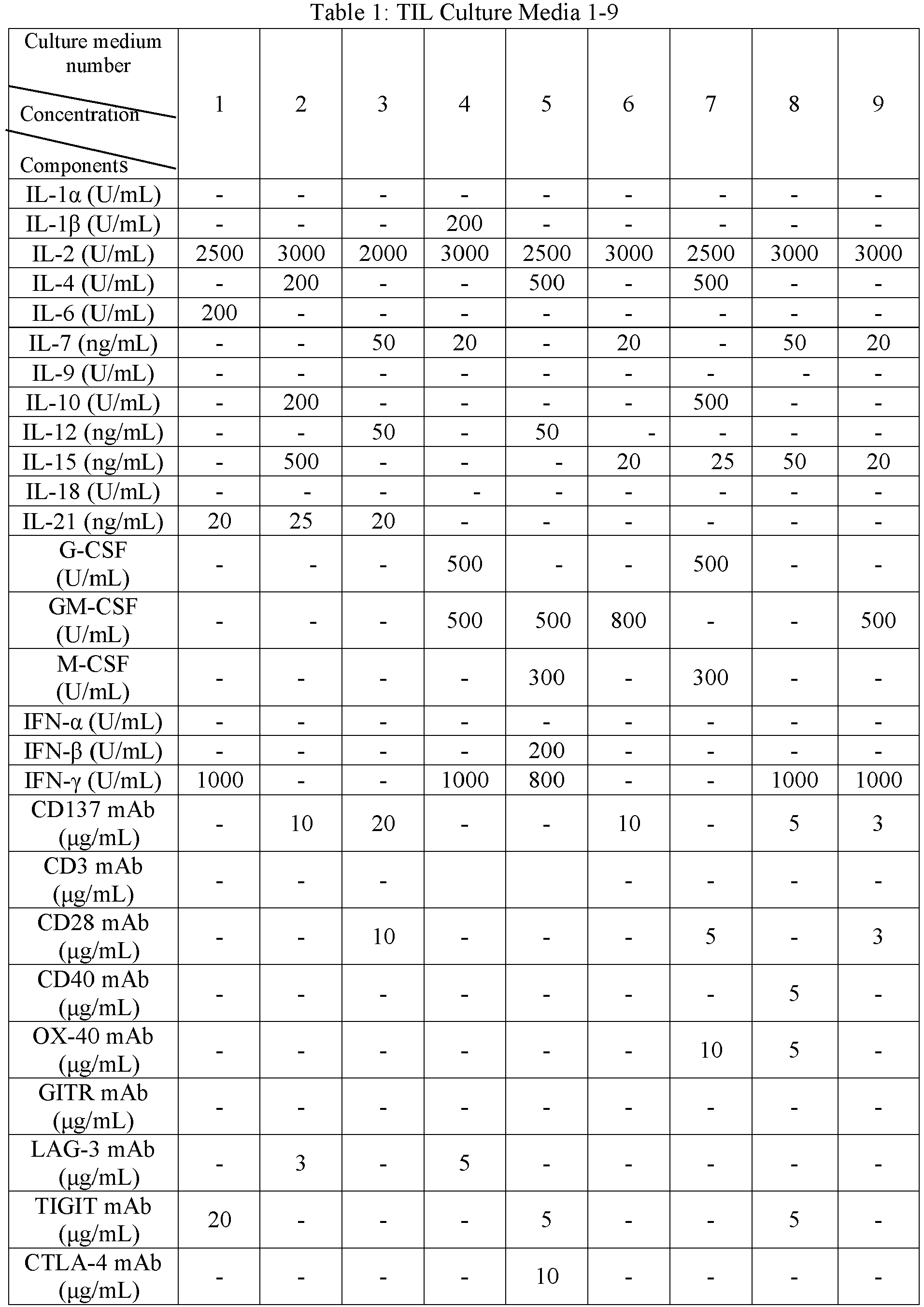

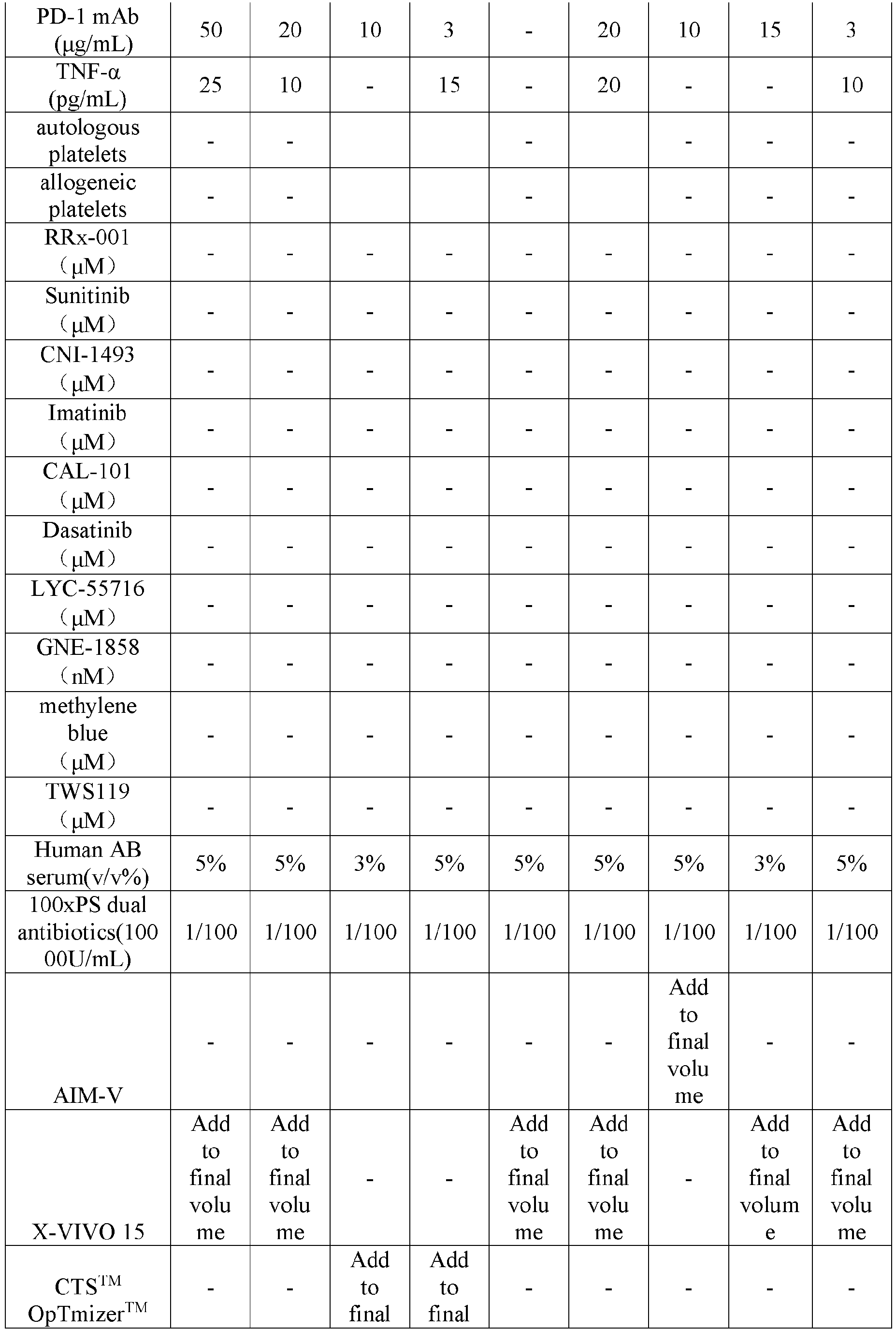

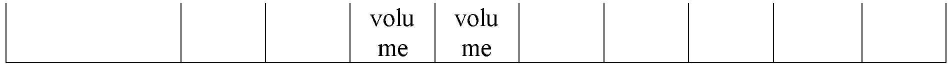

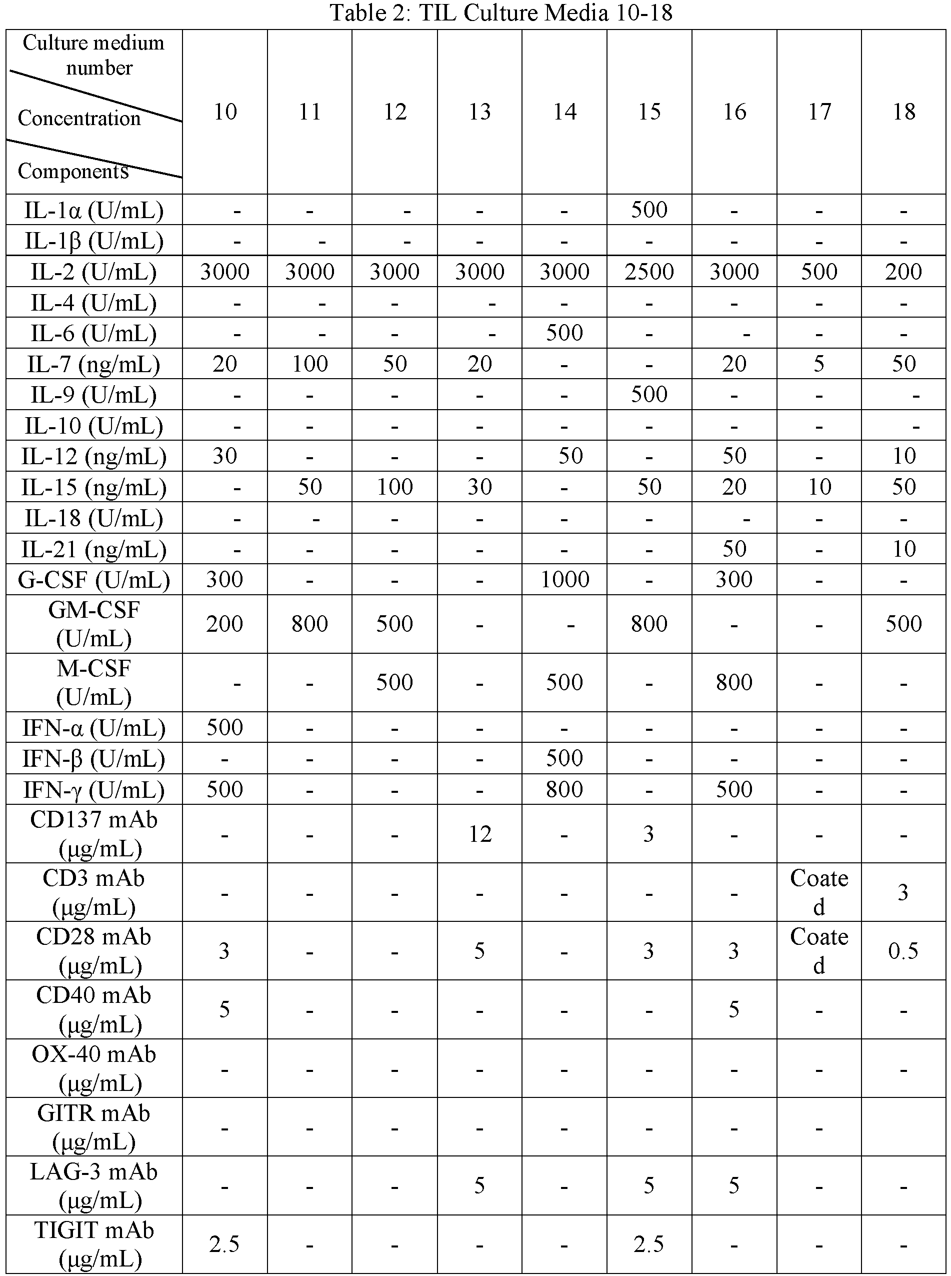

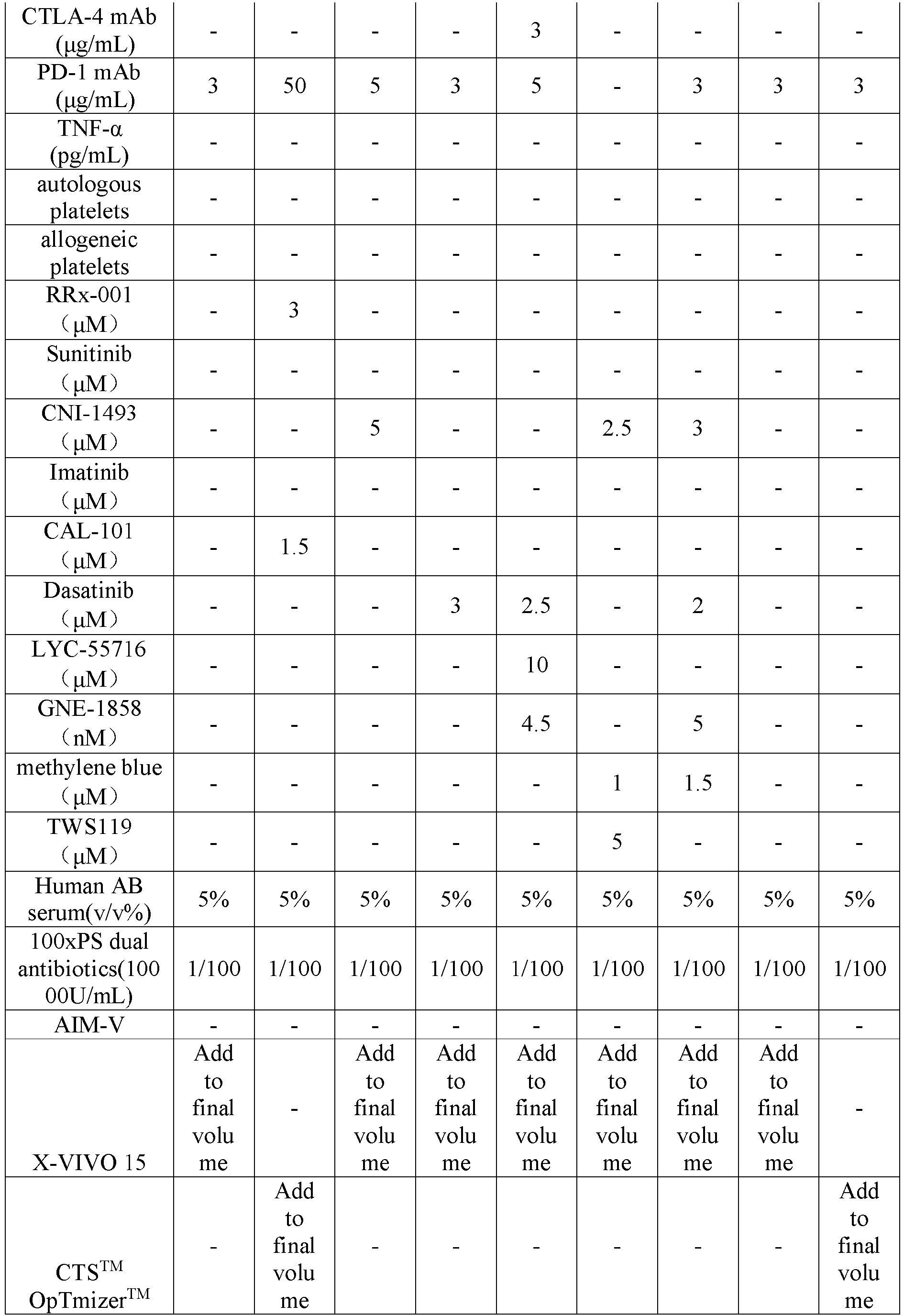

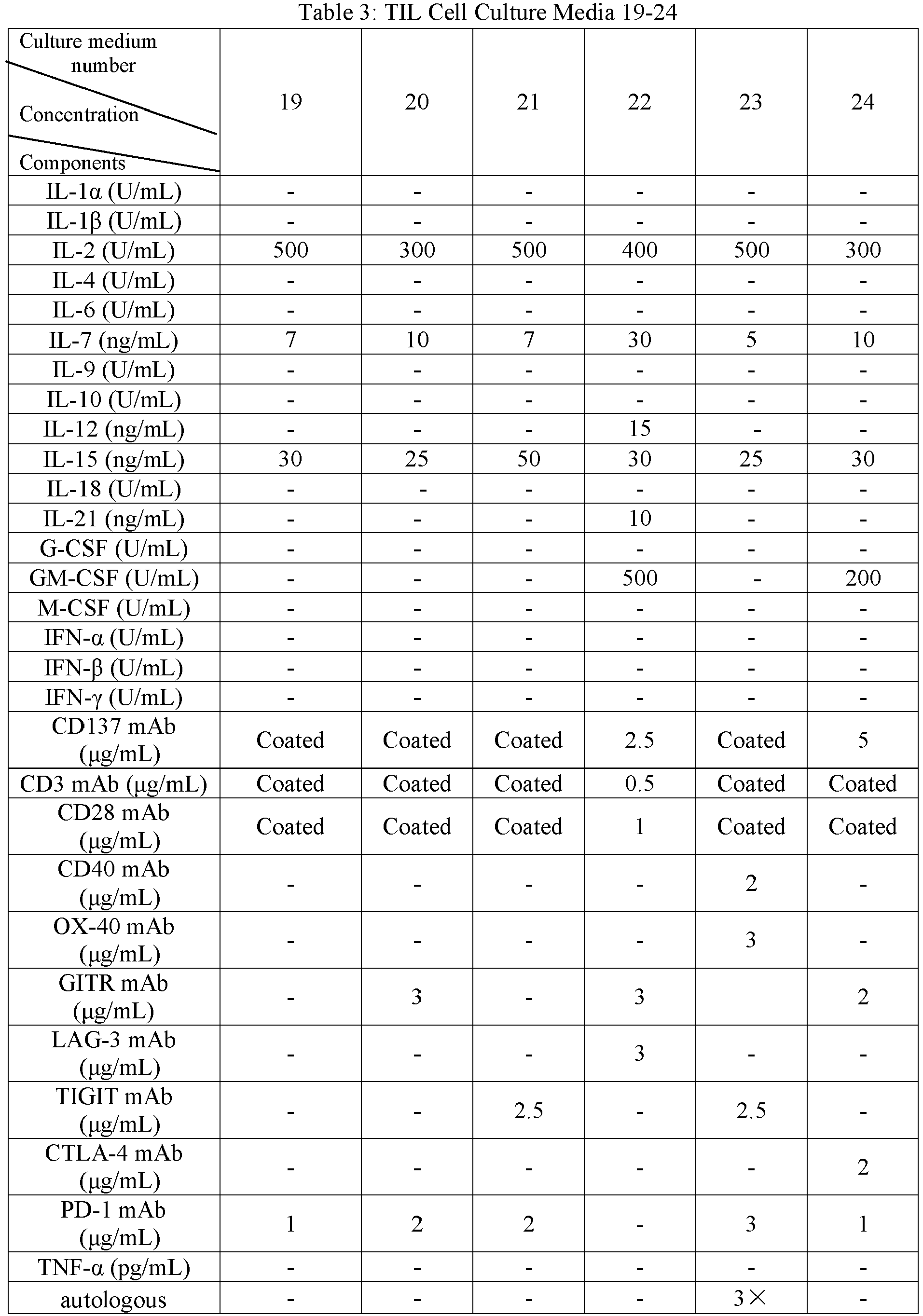

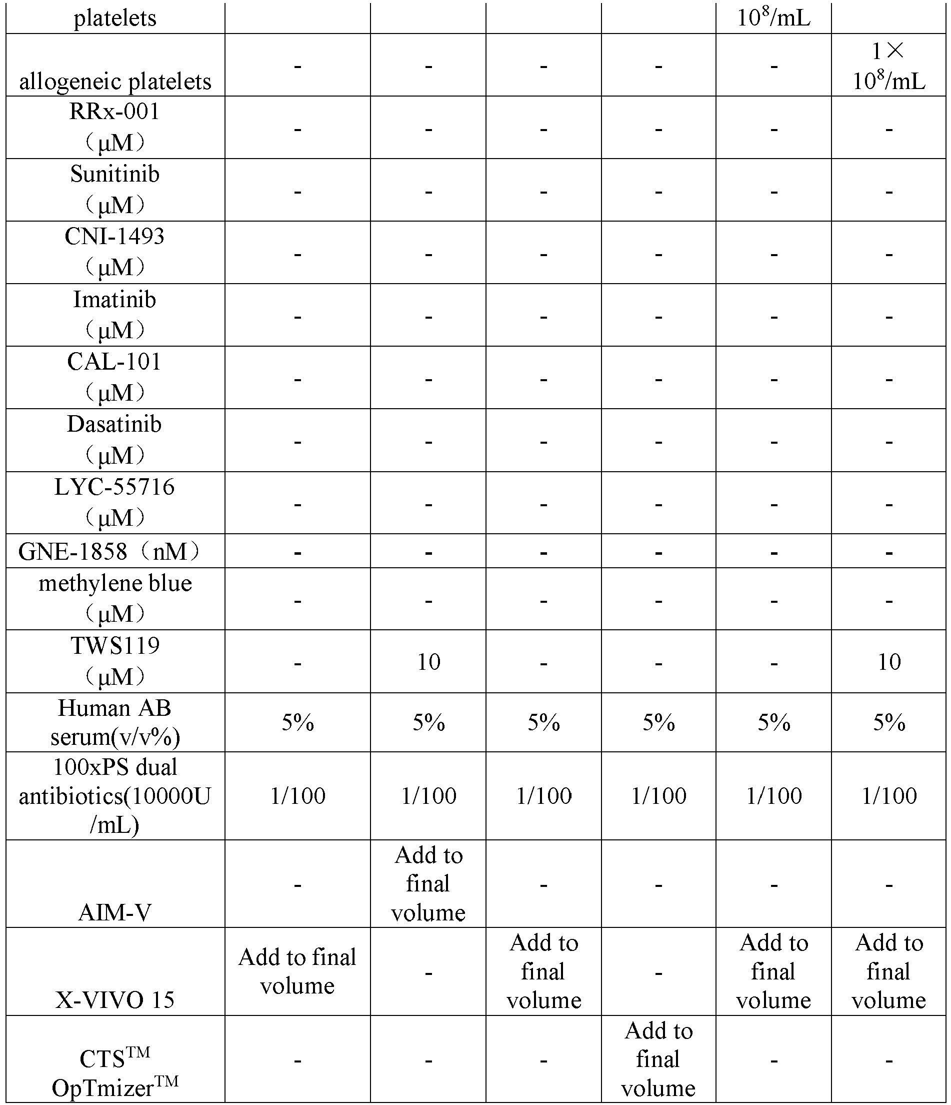

### Example 2, Processing of Patient Solid Tumor Samples and TII, Culture

1) Physiological saline was prepared for use, which contained final concentrations of 100 U/mL of penicillin, 100 µg/mL of streptomycin and 50 µg/mL of gentamicin;
2) In a sterile environment of a BSL-2 cabinet, the freshly tumor tissue samples isolated from tumor patients were placed and washed in a 10 cm petri dish to which 30 mL of physiological saline prepared in step 1) has been added, then transferred to a new 10cm petri dish to which 30mL of physiological saline prepared in step 1) has been added, and washed for 3 times;
3) Adipose tissue and necrotic tissue were removed with a sterile scalpel blade. The tumor tissues were cut into small fragments with a diameter of 3×3×3mm³, two G-REX100 culture tanks (purchased from Wilsonwolf) were prepared, and 42 randomly selected tumor tissue fragments were placed in each G-REX100 culture tank. One of the TIL culture media prepared in example 1 was added to the culture tank as the seed cell culture medium; the excess tumor tissue fragments were cryopreserved with cryopreservant CryoStor10 (purchased from BioLifeSolutions) in liquid nitrogen by a controlled-rate freezer;
4) After 1 L of seed cell culture medium was added to the G-REX100 culture tank containing tumor tissue fragments of 3), the tumor tissue fragments were cultured at 37°C with 5% CO₂. Half of the old seed cell culture medium was removed every 4 days, and same volume of the fresh seed cell medium was added, and TIL seed cells were centrifuged and harvested on the 11th-15th day, then total number of cells were counted and viability measured;
5)
   a: For the expansion media (corresponding to media of No.18 and 22 in Table 2-Table 3) in which all components are medium solution components, 5 L of the pre-warmed expansion medium was added to the G-REX500M culture tank (purchased from Wilsonwolf), the seed cells obtained in step 4) were inoculated at a seeding density of 2.0×10⁵/cm²∼5.0×10⁵/cm², and cultured at 37° C with 5% CO₂. After cell counting was performed every 4 days, half of the old expansion medium was removed and the same volume of fresh expansion medium was added. After the total number of cells in each G-REX500M tank reached 1.0× 10¹⁰, the cells were aliquoted to multiple tanks at the ratio of 1:2, 5L of fresh expansion medium was added and cell culture continued. The cells were cultured in the expansion medium for a total of 12-15 days and harvested, and product TILs were thus obtained;
   b:For the expansion medium containing some components which have been coated in advance as described above (corresponding to the media of No.17, 19, 20, 21, 23, 24 in Table 2-Table 3), the seed cells harvested in 4) were collected and re-suspended to 1.0 × 10⁵/mL∼1.0×10⁶/mL with the expansion medium containing all other components in the corresponding solution except for the coating components, added to the cell culture vessel pretreated with the coating components, and activated at 37°C with 5% CO₂ for 2-3 days. Then the activated cells were collected by centrifugation and inoculated into a G-REX500M culture tank with pre-warmed expansion medium. The expansion medium in the culture G-REX500M tank is the same expansion medium containing all other components in the corresponding solution except for the coating components. The expansion medium volume in each G-REX500M is 5L. The activated seed cells were inoculated at the seeding density of 2.0×10⁵/cm²~5.0×10⁵/cm², and cultured at 37° C with 5% CO₂. After the cells were counted every 4 days, half of the old expansion medium was discarded, fresh expansion medium of and the same volume was added. When the total number of cells in each G-REX500M tank reached 1.0× 10¹⁰, the cells were aliquoted into multiple tanks at the ratio of 1:2, 5L of fresh expansion medium was added to each tank, and cell culture continued. The cells were cultured in the expansion medium in the G-REX500M culture tank for a total of 10-12 days before they were harvested, and product TILs were thus obtained;
6) The phenotypes of the product cells obtained in 5) were detected by flow cytometer, and the IFN-gamma secretion level was measured by HTRF IFN-gamma detection kit (Cisbio Human IFN gamma kit, catalog number: 62HIFNGPET) according to the method described in the instruction.

The following table 4 shows tumor tissue sample numbering, cancer type, seed cell culture medium and expansion medium used in the culture process corresponding to the numbering in Table 1-Table 3, the respective culture time of each tissue sample-matched seed cell culture medium and expansion culture medium and G-REX500M cell seeding density; Table 5 shows the types of coated vessels, re-suspended cell density for coated activation and coating activation time of the corresponding tumor tissue samples in step 5) b.

**Table 4: TII,-derived tissue sample information and the corresponding number of the medium used**

| | Cancer type | Seed cell mediu m No. | Culture time of the seed cell medium (days) | Expan -sion medium No. | Cell seeding density in G-REX500M (cells/cm²) | Culture time of the expansion medium (days) |
|---|---|---|---|---|---|---|
| T001 | gastric cancer | 12 | 12 | 17 | 2.5×10⁵ | 12 |
| T002 | intestinal metastasis of melanoma | 14 | 12 | 20 | 2.5×10⁵ | 12 |
| T003 | cervical cancer | 5 | 12 | 19 | 2.0×10⁵ | 12 |
| T004 | gastric cancer | 11 | 11 | 21 | 2.5×10⁵ | 12 |
| T005 | gastric cancer | 2 | 12 | 23 | 2.5×10⁵ | 11 |
| T006 | intestinal cancer | 8 | 12 | 19 | 2.5 × 10⁵ | 10 |
| T007 | ovarian cancer | 6 | 14 | 18 | 3.0×10⁵ | 15 |
| T008 | cervical cancer | 7 | 12 | 24 | 2.5×10⁵ | 12 |
| T009 | bladder metastasis of recurrent cervical cancer | 9 | 12 | 19 | 2.5×10⁵ | 12 |
| T010 | ovarian metastases of gastric cancer | 15 | 11 | 20 | 4.5 × 10⁵ | 12 |
| T011 | glioma | 1 | 15 | 17 | 5.0×10⁵ | 12 |
| T012 | cervical cancer (recurrent) | 3 | 12 | 23 | 2.5 × 10⁵ | 12 |
| T013 | melanoma | 16 | 13 | 24 | 2.5×10⁵ | 12 |
| T014 | cervical cancer | 4 | 12 | 20 | 2.5×10⁵ | 12 |
| T015 | lymphatic metastasis of endometrial cancer | 10 | 12 | 22 | 2.5×10⁵ | 15 |
| T016 | lymph node metastasis of intestinal cancer | 13 | 12 | 21 | 2.5×10⁵ | 12 |
| T017 | ovarian cancer | 9 | 12 | 20 | 2.5×10⁵ | 12 |
| T018 | pancreatic cancer | 9 | 12 | 19 | 2.5×10⁵ | 12 |

**Table 5: Coated stimulation conditions of TIL seed cells of some of the samples**

| | Cancer type | Types of Coated Vessels | Activated re-suspended cell density | Activating time(days) |
|---|---|---|---|---|
| | | | (/mL) | |
| T001 | gastric cancer | cell culture bag | 2.0×10⁵ | 2 |
| T002 | intestinal metastases of melanoma | cell culture bag | 2.5×10⁵ | 3 |
| T003 | cervical cancer | cell culture bag | 1.0×10⁵ | 3 |
| T004 | gastric cancer | 245mm cell culture dish | 5.2×10⁵ | 3 |
| T005 | gastric cancer | cell culture bag | 2.5×10⁵ | 3 |
| T006 | bowel cancer | cell culture bag | 2.5×10⁵ | 3 |
| T008 | cervical cancer | cell culture bag | 2.5×10⁵ | 3 |
| T009 | bladder metastasis of recurrent cervical cancer | cell culture bag | 2.5×10⁵ | 3 |
| T010 | ovarian metastasis of gastric cancer | cell culture bag | 2.5×10⁵ | 2 |
| T011 | glioma | 245mm cell culture dish | 1.0×10⁶ | 3 |
| T012 | cervical cancer (recurrent) | cell culture bag | 3.5×10⁵ | 2 |
| T013 | melanoma | cell culture bag | 2.5×10⁵ | 3 |
| T014 | cervical cancer | 245mm cell culture dish | 2.5×10⁵ | 3 |
| T016 | lymph node metastasis of intestinal cancer | 245mm cell culture dish | 2.5×10⁵ | 2 |
| T017 | ovarian cancer | cell culture bag | 2.5×10⁵ | 2 |
| T018 | pancreatic cancer | cell culture bag | 2.5×10⁵ | 2 |

245mm cell culture dishes were purchased from Corning (Cat.431111), and cell culture bags were purchased from Takara (CultiLife^{™}215 Culture Bag, Cat.FU0005). The whole process of preparation of the above-mentioned product TILs was carried out in a GMP-level workshop in accordance with the requirements of relevant regulations. After harvest cells were centrifuged, washed with D-PBS, and re-suspended in normal saline. The cells passed the quality control tests (CD3+ ratio, CD4+ and CD8+ ratio, IFN-gamma secretion level, sterility, endotoxin, osmotic pressure, etc.) and all the indicators met the requirements of the "Pharmacopoeia of the People's Republic of China" or the corresponding general standards of immune cell therapy products that have been marketed globally before they were released.

### Example 3, the killing effect of the pharmaceutical composition on the tumor tissue of the patient-derived xenograft (PDX)

### Experimental animals

Immunodeficient B-NDG mice (purchased from Biocytogen) were used as experimental animals to establish PDX models. Hydroxychloroquine used in the experiment was purchased from MCE (Cat.No.: HY-W031727).

Experimental design and grouping were shown in Table 6 as below:

**Table 6: PDX Mice dosing regimen and grouping**

| Group | Drug | Animal number | Administration route | Frequency of administration | Dosage | Time (days) |
|---|---|---|---|---|---|---|
| 1 | PBS | 8 | tail vein | once | 200µL | 40 |
| 2 | TIL | 8 | tail vein | once | 2×10⁷ /200µL | 40 |
| 3 | TIL + hydrox ychloro quine | 8 | Tail vein + intraperito neal injection | once + q. d. | 2×10⁷ /200µL+60mg/k g/day | 40 |

TILs were product TILs derived from the tissue sample T008 prepared in Example 2. Before tail vein injection, the cells were centrifuged and re-suspended in PBS to prepare a PBS cell suspension with a cell density of 1 × 10⁸/mL.

### Animal husbandry

The required number of B-NDG mice were purchased and housed in an SPF-level experimental animal room for an acclimatization period of 7-10 days.

Environment: mice will be housed in transparent resin plastic cages in the animal house. Cage litter is autoclaved wood chips and corncob litter, which are replaced periodically. The animal house is equipped with high-efficiency air filters and will maintain at a temperature between 20-26°C (68-79°F) with a relative humidity of 40-70%. Temperature and humidity are monitored and recorded continuously. The illumination condition is 12 hours of daylight lamp illumination and 12 hours of darkness.

Food and drinking water: Experimental mice can are allowed to access special mouse food (sterilized by irradiation, SLAC Laboratory Animal, Shanghai, China) and sterilized clean drinking water *ad libitum.*

### Establishment of PDX model

1) Treatment of tumor tissue samples from patients: a portion of T008 tumor tissue was used, and necrotic tissue, adipose tissue and connective tissue, etc. were removed from the tumor tissue in sterile condition. After wash, the tumor tissue was dissociated into a number of 5×5×5mm³ tissue fragments with a scalpel, put into the tumor sample-transportation preservation solution UW, and the tumor fragments were ready to be inoculated into B-NDG mice;
2) Tumor tissue sample inoculation: a number of B-NDG mice were used. After the skin of the mouse scapula were prepared, the mouse was fixed with a mouse subcutaneous tumor inoculation fixer, disinfected with iodophor, locally anesthetized with lidocaine, and the tumor fragments of 1) was inoculated to the right groin of the mice by the PDX model tumor fragments inoculation trocar. The day of inoculation was recorded as P0, and the tumor was measured twice a week. The formula for calculating tumor volume is: V=0.5×a×b², where a and b are the long and short diameters of the tumor, respectively;
3) PDX tissue passaging: the growth of tumor tissue in each inoculated mouse was observed. When the volume of tumor tissue reached more than 300mm³, the mice were anesthetized, the tumor fragments taken out, and cut into 5×5×5mm³ tissue fragments with a scalpel under sterile conditions. Then step 2) was repeated, tissue fragments were inoculated into the right groin of a new mouse, and the next generation of PDX tumor began to grow;
4) Step 3) was repeated, and after 2-3 passages, some PDX tissues in the mice were sampled for tissue section pathological analysis. Upon confirming the human nature of the PDX tissues (not murine), they were used to inoculate into 1.5 times the number of mice according to the experimental plan in Table 9 (i.e., 24 mice inoculated with PDX tissue fragments). The tumorigenicity in mice was monitored, and the tumor was measured twice a week until tumor began to form.

### Animal grouping and dosing

When the tumor volume of PDX-inoculated mice reached ~50 mm³, 24 animals with appropriate tumor volume were selected from 36 animals, and randomly grouped according to tumor volume, n=8, to ensure that all groups were comparable at baseline. The grouping day was recorded as D0, and the dosing was carried out according to the scheme in Table 6. During the experiment, the body weight and tumor volume of the animals were measured 3 times a week, and the clinical symptoms of the animals were monitored daily. The tumor volume is expressed in mm³, and the formula used to calculate tumor volume is the same as above.

The tumor tissues of T008 and T018 samples were used for model establishment and the dosing was performed according to the above-mentioned regimen, respectively. TILs in the administered pharmaceutical composition were those prepared in Example 2 which matched their own syngeneic tissue, respectively. The results were observed after 40 days.

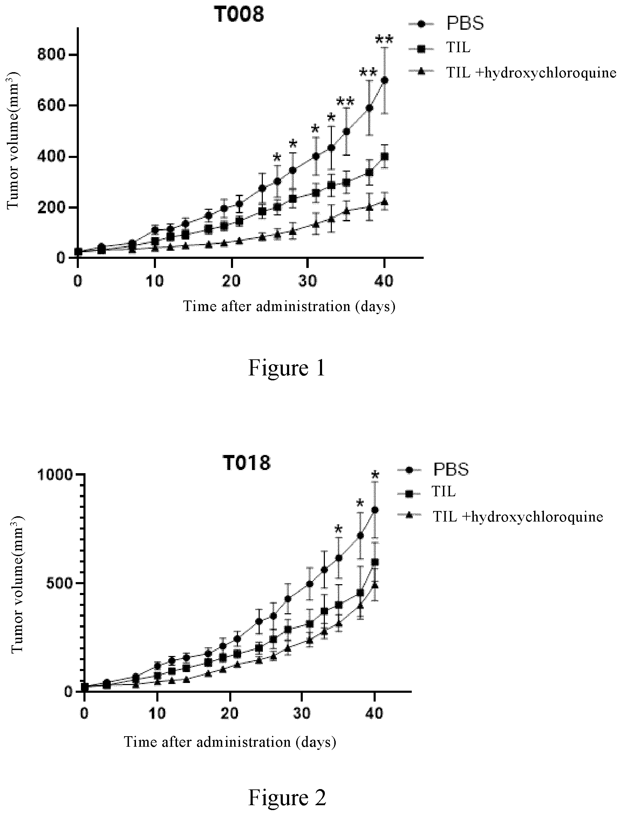

The results are shown in Figure 1 and Figure 2, respectively. Results are analyzed by two-way ANNOVA. ^{∗}: p<0.05; ^{∗∗}: p<0.01. Figure 1 and Figure 2 show the *in vivo* therapeutic effects of the pharmaceutical composition of the present invention on tumor tissue PDX mouse models of T008 and T018, respectively. The results show that after the PDX mouse models of different cancer tissue sources of T008 and T018 were dosed with their respective matched TILs or the pharmaceutical composition of the application, the tumor growth can be significantly inhibited compared with the PBS group. The inter-group differences among the groups of PDX model mice derived from both tissues T008 and T018 are statistically significant. The difference between the TIL group and the TIL +hydroxychloroquine (i.e. the pharmaceutical composition of the present invention) of the T008 model is greater than the that of the T018 model. The above results show that the pharmaceutical composition of the present invention has excellent tumor killing effects on both T008 tissue-derived and T018 tissue-derived PDX tumor models.

### Example 4, the effect of hydroxychloroquine treatment on MHC-I expression of tumor cells of mouse PDX model

PDX mouse models derived from tissue T008 and tissue T018 were established according to the method of example 3. The model animals were grouped according to the following Table 7 in which mice were treated with hydroxychloroquine alone. Hydroxychloroquine used in the experiment was purchased from MCE (Cat.No.: HY-W031727).

**Table 7: Single-drug dosing regimen of hydroxychloroquine and grouping in PDX mice**

| Group | Drug | Animal number | Administra tion route | Dosing Frequency | Dosage | Time (days) |
|---|---|---|---|---|---|---|
| 1 | PBS | 8 | tail vein | q.d. | 200µL | 30 |
| 2 | Hydroxychloro quine | 8 | intraperito neal injection | q.d. | 60mg/kg/d ay | 30 |

Mice were sacrificed 30 days after treatment, and tumor tissues were taken out and digested into a single-cell suspension with dispase. After staining with a general HLA-A, B, C flow cytometric antibody (Biolegend, PE-anti-human HLA-A, B, C Antibody Cat.NO.311406), the phenotypes and fluorescence intensity (MFI) were detected by flow cytometry, and the MFI values of different groups of HLA-type I positive cells were counted and compared.

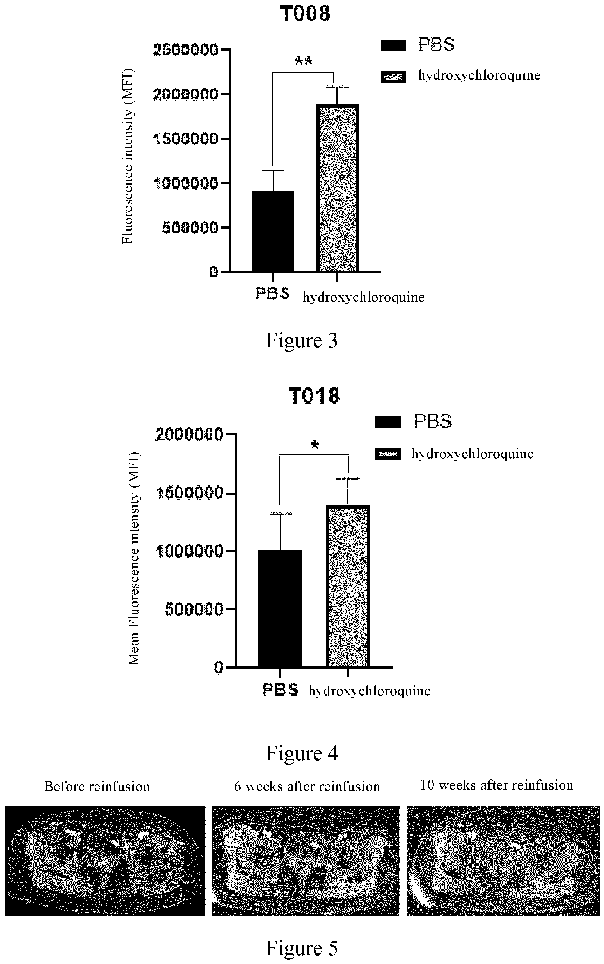

The results are shown in Figure 3 and Figure 4. Compared with the control group treated with PBS, the expression levels of type I HLA on the surface of tumor cells in mice with PDX tissues derived from T008 and T018 were significantly increased after being treated with hydroxychloroquine with a statistical difference. The above results show that hydroxychloroquine treatment of tumor tissue produces a significant effect on the promotion of type I HLA expression on the surface of tumor cells derived from T008 and T018 tissues.

### Example 5, Clinical application of the pharmaceutical composition containing TIL and hydroxychloroquine

In order to validate the clinical safety and effectiveness of the pharmaceutical composition containing the TILs obtained by the culture method of the present application and hydroxychloroquine, a clinical trial of TIL infusion therapy for solid tumors was carried out. This clinical trial had been approved by the Ethics Committee of Shanghai Tenth People's Hospital. For details, please refer to http://www.chictr.org.cn/, registration number: ChiCTR2100044705; or refer to www.clinicaltrials.gov, registration number is NCT04766320. In addition, this trial also involved cooperation with Tong Ren Hospital Shanghai Jiaotong University School of Medicine and the Second Affiliated Hospital of Soochow University, www.clinicaltrials.gov with registration numbers being NCT04967833 and NCT04943913, respectively.

### Study plan

### 1. Inclusion and exclusion criteria and grouping methodology of test subjects

Inclusion criteria:
1) 18 years old <, age <, 75 years old;
2) Patients pathologically diagnosed with primary/recurrence/metastasis of malignant tumors;
3) Life expectancy > 3 months;
4) Karnofsky ≥ 60% or ECOG scoring 0-2 points;
5) The subject currently failed in standard treatment, or had no standard treatment plan;
6) The subject must have a tumor area or malignant effusion suitable for puncture or resection of living tissue from which TILs can be isolated;
7) There was at least one assessable tumor lesion;
8) White blood cell count ≥ 2.5 × 10⁹/L, absolute neutrophil count ≥ 1.5 × 10⁹/L, platelet count ≥ 100x 10⁹; hemoglobin ≥90g/L;
9) Serum creatinine clearance rate was 50 mL/min or higher; creatinine ≤ 1.5 × ULN; ALT (alanine aminotransferase)/AST (aspartate aminotransferase) < 3 times of the normal group, ALT/AST of those with liver metastases < 5 times of the normal group; Total bilirubin≤ 1.5 × ULN;
10) Activated partial thromboplastin time (APTT) ≤ 1.5 × ULN; International Normalized Ratio (INR) ≤ 1.5×ULN;
11) Sufficient venous access, no absolute or relative contraindications for surgery or puncture;
12) Subjects with reproductive potential must be willing to implement the approved high-efficiency contraceptive method at the time of signing informed consent, and continue to implement it within one year after the completion of the lymphodepletion regimen;
13) Any treatment for malignant tumors, including radiotherapy, chemotherapy and biologicals, must be stopped 28 days before the TILs are obtained;
14) The subject has sufficient comprehensive capability and voluntarily signs the informed consent;
15) The subject has good compliance and be able to adhere to the study visit plan and other protocol requirements.

Exclusion criteria:
1) Need to use glucocorticoid therapy, with a daily dose of prednisone greater than 15 mg (or equivalent dose of hormone);
2) Require immunosuppressive therapy against autoimmune diseases;
3) Serum creatinine>1.5×ULN; serum glutamic-oxaloacetic transaminase (SGOT)>5 times of the upper limit of normal value; total bilirubin>1.5×ULN;
4) Forced expiratory volume in the 1st second (FEV1) < 2L, diffusing capacity of lung carbon monoxide (DLCO) (corrected) < 40%
5) The subject had significant cardiovascular abnormality according to any one of the following definitions: New York Heart Association (NYHA) class III or IV congestive heart failure, clinically significant hypotension, clinically uncontrolled hypertension, uncontrolled symptomatic coronary arteries disease, or ejection fraction <35%; severe cardiac rhythm or conduction abnormalities, such as ventricular arrhythmia requiring clinical intervention, degree II-III atrioventricular block, etc.;
6) The subject has human immunodeficiency virus (HIV) infection or HIV antibody test positive, active hepatitis B or hepatitis C virus infection (HBsAg positive and/or anti-HCV positive), syphilis infection or treponema pallidum antibody positive;
7) Severe physical or mental illness;
8) Imaging evidence of positive blood culture or infection;
9) Receiving other medicaments, or other biological, chemotherapy or radiotherapy currently or within one month;
10) The subject has a history of allergic reactions due to chemical or biological composition compounds similar to cell therapy;
11) The subject has received immunotherapy and manifests irAE grade ≥ 3;
12) The adverse reactions of the previous anti-tumor therapy has not recovered to CTCAE version 5.0 grade evaluation <, grade 1 (except for toxicities that the investigator judges to have no safety risk such as alopecia);
13) Pregnant or lactating women;
14) The investigator believes that the subject has a history of other serious systemic diseases, or is not suitable for participating in this clinical study for other reasons.

### Evaluation Criteria for Efficacy

Main efficacy indicators:
1) Objective response rate (ORR);
2) Disease Control Rate (DCR);
3) Duration of response (DOR);
4) Progression-free survival (PFS);
5) Overall survival (OS).

Secondary efficacy indicators:
1) Clinical efficacy evaluation: complete remission (CR), partial remission (PR), Stable disease (SD), Progressive disease (PD), etc.;
2) Changes in quality of life before and after treatment.

Efficacy was defined as observable ORR according to the criteria of RECIST1.1.

### 3. Records of adverse events and countermeasures

### 3.1 Definition of Adverse Events

Adverse events (AE): Adverse medical events that occurred from the time of the subject signed the informed consent and was enrolled in the trial, to the last medical follow-up, which can be manifested as symptoms and signs, diseases or abnormal laboratory tests, but was not necessarily related to a causal relationship with the treatment or experimental medicament.

A new condition or deterioration of a pre-existing condition was considered as an AE. Stable chronic diseases that were present before entry into study and did not worsen during the study period, such as arthritis, were not considered as an AE. Abnormal laboratory test results, clinical symptoms or signs judged by the investigator to be clinically significant were considered as an AE.

Serious Adverse Event (SAE): Refers to adverse events that meet one or more of the following conditions during the trial: (1) leading to death; (2) life-threatening, which means that serious patients were at risk of immediate death, not hypothetical death may occur in severe cases in the future; (3) leading to hospitalization or prolonged hospitalization; (4) permanent or significant loss of function; (5) teratogenic, birth defects; (6) other important medical events.

### 3.2. Criteria for judging the severity of adverse events

The severity of all adverse events that occurred during the study was evaluated according to NCI-CTCAE version 5.0 and categorized into grades 1 to 5:
Grade 1: Mild; asymptomatic or mild; only clinical or diagnostic findings; no treatment required.
Grade 2: Moderate; minor, topical, or noninvasive treatment required; age-appropriate limitation in instrumental activities of daily living.
Grade 3: Severe or medically significant but not immediately life-threatening; resulting in hospitalization or prolonged hospitalization; disabling; limiting personal activities of daily life.
Grade 4: Life-threatening; urgent treatment required.
Grade 5: AE-related death.

### 4. Pretreatment before infusion

Before the infusion of TILs, the patient received a lymphocyte preconditioning regimen (hydroxychloroquine 600-800 mg×1 day, cyclophosphamide 20-25 mg/kg/d×3 days), the drugs, dosages and number of days (dosing frequency) used in the preconditioning regimen will be adjusted by the investigator according to the actual condition of the patient. The cyclophosphamide and hydroxychloroquine administered to the subjects were provided by the hospital.

The day of infusion was defined as Day 0. Before the infusion of TILs, the investigator needs to perform the following chemotherapy pretreatment on the patient (the patient will experience leukopenia after pretreatment, and infection should be prevented, such as avoiding unnecessary personnel mobility in the ward, and patients wearing masks, etc.):
Day -5:intravenous injection of cyclophosphamide (Cy) 20-25 mg/kg + oral administration of hydroxychloroquine (HCQ) 600-800 mg
Day -4: intravenous injection of cyclophosphamide 20-25 mg/kg
Day -3: intravenous injection of cyclophosphamide 20-25 mg/kg
Day -2 and Day -1:the patient rest
Day 0, infusion of TILs.

### 5. TIL infusion and therapeutic efficacy evaluation

The patients were infused on Day 0 after the pretreatment prior to the infusion in the aforementioned 4 was completed. Each patient received a single infusion, and the dose of one infusion was 2.0×10¹⁰-5.0×10¹⁰ TILs, the cell density was 1.0×10⁸/mL~2.0× 10⁸/mL, and the volume of the infused cell preparation was 200mL-600mL. The patients were infused with TILs on Day 0, and no cytokines were administered post-infusion. After infusion, the patient were required to stay in hospital for 5-8 days for observation, and patient's conditions after infusion were monitored and recorded. With the patient's informed consent, peripheral blood was drawn from the patient at different time points to detect the composition and changes of PBMCs, and the efficacy was evaluated by imaging 1-3 months after the infusion.

### 6. Results of TIL clinical infusion

A total of 16 subjects had been enrolled so far. Table 8 as shown below records the basic conditions of 11 patients who had undergone clinical efficacy evaluation (evaluation time varies within 1-3 months after infusion):

**Table 8: Comprehensive information on clinical medication of some tumor patients**

| Serial number | Tumor type | Number of the infused TILs | Pretreatment prior to infusion | Clinical efficacy |
|---|---|---|---|---|
| T009 | cervical cancer | 2.0×10¹⁰ | Cy 20mg/kg+ HCQ 600mg | CR |
| T010 | ovarian metastasis of gastric cancer | 5.0×10¹⁰ | Cy 25mg/kg+ HCQ 600mg | SD |
| T011 | glioma | 2.5×10¹⁰ | Cy 25mg/kg+ HCQ 600mg | PD |
| T007 | ovarian cancer | 2.0×10¹⁰ | Cy 20mg/kg+ HCQ 800mg | SD |
| T012 | cervical cancer | 2.0×10¹⁰ | Cy 20mg/kg+ HCQ 600mg | PR |
| T003 | cervical cancer | 3.5×10¹⁰ | Cy 25mg/kg+ HCQ 600mg | SD |
| T013 | melanoma | 2.0×10¹¹ | Cy 20mg/kg+ HCQ 700mg | SD |
| T014 | cervical cancer | 4.5×10¹⁰ | Cy 20mg/kg+ HCQ 600mg | SD |
| T015 | endometrial cancer | 4.0×10¹⁰ | Cy 25mg/kg+ HCQ 600mg | SD |
| T016 | colorectal cancer | 2.1×10¹⁰ | Cy 20mg/kg+ HCQ 600mg | SD |
| T017 | ovarian cancer | 3.0×10¹⁰ | Cy 25mg/kg+ HCQ 600mg | PR |

Among the 16 subjects, adverse events included grade 1 chills in 5 cases (33.3%), grade 1 fever in 7 cases (46.7%), and grade 2 fever in 6 cases (40%), and all symptoms disappeared over time. No other adverse event was observed.

Patient T017 was evaluated as a partial response (PR) based on the imaging evaluation that the tumor shrank significantly. Peripheral blood was drawn from the patient before and after the infusion of TILs, and the levels of some tumor markers were detected, and the comparison results are shown in Table 9 as below.

**Table 9: Comparison of the values of some tumor markers before and after treatment of patient T017**

| | Carci noe mbry onic antig en(n g/mL ) | Alpha-Fetoprot ein (ng/mL) | CA153 (U/mL ) | CA125 (U/mL ) | CA199 (U/mL ) | human epididymi s protein 4(pmol/m L) | postm enopa usal ROM A value | prem enopa usal ROM A value |
|---|---|---|---|---|---|---|---|---|
| Nor mal range | <5.2 | <7.0 | <26.4 | <35 | <27 | <82.9 | <29.9 % | <43.3 % |
| Before infusio n | 1.07 | 3.8 | 143 | 60.1 | 12.43 | 124 | 48 | 43.3 |
| 30 days after infusio n | 0.71 | 3.81 | 104 | 30.8 | 14.58 | 87.1 | 28.2 | 24 |

The results in Table 9 show that before infusion in patient T017, the above tumor markers significantly exceeded the normal range, except for alpha-fetoprotein and CA199, which were within the normal range. The above tumor markers in the peripheral blood of the patient were tested again 30 days after TIL infusion, and it was found that the several factors that exceeded the normal standard had returned to or remained within the normal range, except for human epididymis protein 4, which was slightly higher than the normal range.

Figure 5 showed that the results of imaging evaluation of patient T009 after being administered the pharmaceutical composition. MRI results show that the size of the tumor lesion before infusion was about 3 cm in diameter. The patient's tumor lesions had shrunk significantly by the 6th week after the TIL infusion (when it was judged as partial response, PR) compared with that before the infusion. After 10 weeks post-dosing, the tumor was no longer detectable by imaging, and it was judged as complete remission (CR) according to the RECIST 1.1 guideline standard.

The above results show that the pharmaceutical composition comprising TILs and hydroxychloroquine of the present invention produces superior clinical efficacy on various solid tumors.

Although specific embodiments of the present invention have been described in detail, those skilled in the art will understand. Based on all the teachings that have been disclosed, various modifications and substitutions can be made to details, and these changes are all within the scope of the invention. The full scope of the invention is given by the appended claims and any equivalents thereof.

## Claims

1. A pharmaceutical composition, comprising a tumor cell killing agent, a compound of Formula (I) or a pharmaceutically acceptable salt, isomer, racemate, solvate, hydrate or prodrug, and a pharmaceutically acceptable excipient, 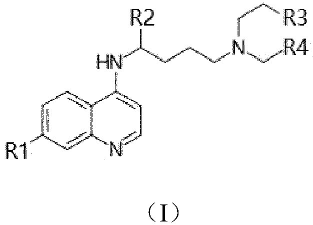
wherein, R1-R4 are each independently selected from the group consisting of H, alkyl, halogen, hydroxyl, amino, and the alkyl is optionally substituted by a group selected from the group consisting of H, C1-C6 alkyl, halogen, hydroxyl, amino, carboxyl,
preferably, each of R1-R4 is independently selected from the group consisting of H, C1-C6 alkyl, halogen, hydroxyl, and said C1-C6 alkyl is optionally substituted by a group selected from the group consisting of H, C1-C6 alkyl, halogen, hydroxyl.

2. The pharmaceutical composition according to claim 1, wherein,
R1 is selected from the group consisting of C1-C6 alkyl, halogen, and/or
R2 is selected from the group consisting of H, C1-C6 alkyl, and/or
R3 is selected from the group consisting of C1-C6 alkyl, halogen, hydroxyl, and/or
R4 is selected from the group consisting of C1-C6 alkyl, halogen, hydroxyl.

3. The pharmaceutical composition according to claim 2, wherein,
R1 is selected from the group consisting of F, Cl, Br, and/or
R2 is selected from the group consisting of C1-C4 alkyl, and/or
R3 is selected from the group consisting of C1-C4 alkyl, hydroxyl, and/or
R4 is selected from the group consisting of C1-C4 alkyl, hydroxyl.

4. The pharmaceutical composition according to any one of claims 1-3, wherein, the pharmaceutically acceptable salt is selected from the group consisting of hydrochloride, sulfate, nitrate, and phosphate.

5. The pharmaceutical composition according to any one of claims 1-3, wherein, the tumor cell killing agent is an immune cell having anti-tumor activity,
preferably, the immune cells having anti-tumor activity is in the form of a cell cryopreservant preparation,
preferably, the cell cryopreservant preparation comprises a cryopreservant,
preferably, the cell density of the cell cryopreservant preparation is 1.0×10⁸ cells /mL~2.0×10⁸ cells /mL,
preferably, the number of immune cells contained in the immune cells having anti-tumor activity is at least 10⁸ cells,
preferably, the immune cells having anti-tumor activity are one or more cells selected from the group consisting of a LAK, a DC, a CIK, a DC-CIK, a CAR-T, a TCR-T, a NK, a CAR- NK, a TIL.

6. The pharmaceutical composition according to claim 5, wherein, the immune cells having anti-tumor activity are TILs, and the TILs are prepared by a method comprising:
(1) incubating a tumor cell-containing tissue with a TIL seed cell culture medium to obtain a first population of TILs,
(2) incubating the first population of TILs with a TIL cell expansion medium to obtain a second population of TILs,
preferably, the method has one or more characteristics selected from the group consisting of:
a) the tumor tissue is pretreated, preferably, the pretreatment includes fragmentation and/or dissociation of the tumor tissue,
b) said tumor cell-containing tissue is tumor tissue or body fluid of a subject having a cancer,
c) the TIL seed cell culture medium described in step (1) includes any one of the following combinations of the components: 1) IL-2, IL-6, IL-21, IFN-gamma, a TIGIT antibody, a PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1 beta, IL-2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M-CSF, a CD28 antibody, an OX-40 antibody, a PD-1 antibody, serum, dual antibiotics and basal medium; 8) IL-2, IL-7, IL-15, IFN-gamma, a CD137 antibody, a CD40 antibody, an OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basic medium; 12) IL-2, IL-7, IL-15, GM-CSF, M-CSF, a PD-1 antibody, CNI-1493, serum, dual antibiotics and basic medium; 13) IL-2, IL-7, IL-15, a CD137 antibody, a CD28 antibody, a LAG3 antibody, a PD-1 antibody, Dasatinib, serum, dual antibiotics and basal medium; 14) IL-2 , IL-6, IL-12, G-CSF, M-CSF, IFN-beta, IFN-gamma, a CTLA-4 antibody, a PD-1 antibody, Dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, Dasatinib, GNE-1858, serum, dual antibiotics and basal medium,
d) the incubation of step (1) lasts at least 5 days,
e) the incubation of step (1) lasts at most 20 days,
f) the temperature of the incubation of step (1) is 30-42°C, preferably 37°C,
g) the concentration of CO2 in the incubation of step (1) is 5%,
h) The TIL cell expansion medium described in step (2) includes any one of the combinations of the following components: 1) 200-6000 IU/mL of IL-2, 5-100 ng/mL of IL-7, 5-100 ng/mL of IL-15, and 3-100 µg/mL of PD-1 antibodies, serum, dual antibiotics and basal medium or the proportional concentration of these components; 2) 200-6000 IU/mL of IL-2, 5-100 ng/mL of IL-7, 5-100 ng/mL of IL-15, and 1-100 µg/mL of PD-1 antibodies, serum, dual antibiotics and basal medium or the proportional concentration of these components; 3) 200-6000 IU/mL of IL-2, 5-100 ng/mL of IL-7, 5-100 ng/mL of IL-15, 3-100 µg/mL PD-1 antibodies, 5-100 ng/mL of IL-21, 5-100 ng/mL of IL-12, 1-10 µg/mL of CD3 antibodies, 1-10 µg/mL of CD28 antibodies and 200-5000 U/mL of GM-CSF, serum, dual antibiotics and basal medium or the proportional concentration of these components; 4) 200-6000 IU/mL of IL-2, 5-100 ng/mL of IL-7, 5-100 ng/mL of IL-15, 3-100 µg/mL of PD- 1 antibodies, 5-100 ng/mL of IL-21, 5-100 ng/mL of IL-12, 1-10 µg/mL of CD3 antibodies, 0.5-10 µg/mL of CD28 antibodies and 200-5000 U/mL of GM-CSF , serum, dual antibiotics and basal medium or the proportional concentration of these components; 5) 200-6000 IU/mL of IL-2, 5-100 ng/mL of IL-7, 5-100 ng/mL of IL-15, 3-100 µg/mL of PD-1 antibodies, 5-100 ng/mL of IL-21, 5-100 ng/mL of IL-12, 1-10 µg/mL of CD3 antibodies, 1-10 µg/mL of CD28 antibodies , 1-100 µg/mL of CD137 antibodies and 1-100 µg/mL of GITR antibodies, serum, dual antibiotics and basal medium or the proportional concentration of these components; 6) 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, 0.5-10 µg/mL of CD3 antibodies, 1-10 µg/mL of CD28 antibodies, 1-100 µg/mL of CD137 antibodies and 1-100 µg/mL of GITR antibodies, serum, dual antibiotics and basal medium or the proportional concentration of these components; 7) 200-6000 IU/mL of IL-2, 5-100 ng/mL of IL-7, 5-100 ng/mL of IL-15, 3-100 µg/mL of PD-1 antibodies, 1-10 µg/mL of CD3 antibodies, 1-10 µg/mL of CD28 antibodies, 1-100 µg/mL of CD137 antibodies, 1-100 µg/mL of GITR antibodies, 200-5000 U/mL of GM-CSF and 1-500 µM of TWS119, serum , dual antibiotics and basal medium or the proportional concentration of these components; 8) 200-6000 IU/mL of IL-2, 5-100 ng/mL of IL-7, 5-100 ng/mL of IL-15, 1-100 µg/mL of LAG-3 antibodies, 5-100 ng/mL of IL-21, 5-100 ng/mL of IL-12, 0.5-10 µg/mL of CD3 antibodies, 0.5-10 µg/mL of CD28 antibodies, 1-100µg/mL of CD137 antibodies and 1-100 µg/mL of GITR antibodies, serum, dual antibiotics and basal medium or the proportional concentration of these components;
h) the incubation of step (2) lasts at least 5 days,
i) the incubation of step (2) lasts at most 20 days,
j) the temperature of the incubation of step (2) is 30-42°C, preferably 37°C,
k) the concentration of CO₂ in the incubation of step (2) is 5%,
1) the tumor cell-containing tissue is a tumor tissues or body fluids of subjects having a cancer selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma,
preferably, the base medium is any one selected from the group consisting of AIM-V, X-VIVO, DMEM, RPMI1640, OpTmizer^{™}, and FUJIFILM Irvin MHM-C,
preferably, the serum is selected from the group consisting of human AB serum, autologous serum or animal-derived serum, and preferably, the concentration of the serum is 1-10%.

7. The pharmaceutical composition according to claim 5, wherein, the immune cells with anti-tumor activity are TILs, and the TILs are prepared by the following methods, comprising:
(1) incubating a tumor cell-containing tissue with a TIL seed cell culture medium to obtain a first population of TILs,
(2) contacting the first population of TILs described in (1) with any one or more of the antibodies coupled to the matrix in the aforementioned culture composition to obtain a second population of TILs,
(3) culturing the second population of TILs described in (2) in a TIL cell expansion culture medium to obtain a third population of TILs,
preferably, the method has one or more characteristics selected from the group consisting of:
a) the tumor tissue is pretreated, preferably, the pretreatment includes fragmentation and/or dissociation of the tumor tissue,
b) said tumor cell-containing tissue is a tumor tissue or body fluid of a subject having a cancer,
c) the TIL seed cell culture medium described in step (1) includes any one of the following combinations of the components: 1) IL-2, IL-6, IL-21, IFN-gamma, a TIGIT antibody, a PD-1 antibody, TNF-alpha, serum, dual antibiotics and basal medium; 2) IL-2, IL-4, IL-10, IL-21, a CD137 antibody, a LAG3 antibody, a PD-1 antibody, TNF-alpha, serum, dual antibiotics and basal medium; 3) IL-2, IL-7, IL-12, IL-21, a CD137 antibody, a CD28 antibody, a PD-1 antibody, serum, dual antibiotics and basal medium; 4) IL-1 beta, IL -2, IL-7, G-CSF, GM-CSF, IFN-gamma, a LAG3 antibody, a PD-1 antibody, TNF-alpha, serum, dual antibiotics and basal medium; 5) IL-2, IL-4, IL-12, GM-CSF, M-CSF, IFN-beta, IFN-gamma, a TIGIT antibody, a CTLA-4 antibody, serum, dual antibiotics and basal medium; 6) IL-2, IL-7, IL-15 , GM-CSF, a CD137 antibody, a PD-1 antibody, TNF-alpha, serum, dual antibiotics and basal medium; 7) IL-2, IL-4, IL-10, IL-15, G-CSF, M-CSF, a CD28 antibody, an OX-40 antibody, a PD-1 antibody, serum, dual antibiotics and basal medium; 8) IL-2, IL-7, IL-15, IFN-gamma, a CD137 antibody, a CD40 antibody, an OX-40 antibody, a TIGIT antibody, a PD-1 antibody, serum, dual antibiotics and basal medium; 9) IL-2, IL-7, IL-15, GM-CSF, IFN-gamma, a CD137 antibody, a CD28 antibody, a PD-1 antibody, TNF-alpha, serum, dual antibiotics and basal medium; 10) IL-2, IL-7, IL-12, G-CSF, GM-CSF, IFN-alpha, IFN-gamma, a CD28 antibody, a CD40 antibody, a TIGIT antibody, a PD-1 antibody, TNF-alpha, serum, dual antibiotics and basal medium; 11) IL-2, IL-7, IL-15, GM-CSF, a PD-1 antibody, RRx-001, CAL-101, serum, dual antibiotics and basic medium; 12) IL-2, IL-7, IL-15, GM-CSF, M-CSF, a PD-1 antibody, CNI-1493, serum, dual antibiotics and basic medium; 13) IL-2, IL-7, IL-15, a CD137 antibody, a CD28 antibody, a LAG3 antibody, a PD-1 antibody, Dasatinib, serum, dual antibiotics and basal medium; 14) IL-2 , IL-6, IL-12, G-CSF, M-CSF, IFN-beta, IFN-gamma, a CTLA-4 antibody, a PD-1 antibody, Dasatinib, LYC-55716, GENE-1858, serum, dual antibiotics and basal medium; 15) IL-1 alpha, IL-2, IL-9, IL-15, GM-CSF, a CD137 antibody, a CD28 antibody, a LAG3 antibody, a TIGIT antibody, CNI-1493, serum, dual antibiotics and basal medium; 16) IL-2, IL-7, IL-12, IL-15, IL-21, G-CSF, M-CSF, IFN-gamma, a CD28 antibody, a CD40 antibody, a LAG3 antibody, a PD-1 antibody, CNI-1493, Dasatinib, GNE-1858, serum, dual antibiotics and basal medium,
d) the incubation of step (1) lasts at least 5 days,
e) the incubation of step (1) lasts at most 20 days,
f) the temperature of the incubation of step (1) is 30-42°C, preferably 37°C,
g) the concentration of CO₂ in the incubation of step (1) is 5%,
h) the matrix described in step (2) is a multi-well plate, a cell culture dish or a cell culture bag,
i) the coupling described in step (2) is covalent coupling or non-covalent coupling,
j) the activating antibody coupled to the matrix in step (2) includes any one or more of a CD3 antibody, a CD28 antibody and a CD137 antibody; preferably, includes a CD3 antibody and a CD28 antibody; more preferably, includes a CD3 antibody, a CD28 antibody and a CD137 antibody,
k) the contacting in step (2) is to incubate the first population of TIL cell in step (1) with the antibody coupled to the matrix; preferably, the temperature of the incubation is 30-42°C, preferably 37°C; preferably, the concentration of CO₂ of the incubation is 5%,
l)in step (2), the incubation is carried out in a medium containing the combination of components selected from the group consisting of:
1) 200-500 IU/mL of IL-2, 5-50 ng/mL of IL-7, 5-50 ng/mL of IL-15, 1-50 µg/mL of PD-1 antibodies, 200-1000 U/mL of GM-CSF, serum, dual antibiotics, and basal medium or the proportional concentration of these components; 2) 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, 1-100 µg/mL of CD137 antibodies and 1-100 µg/mL of GITR antibodies, serum, dual antibiotics and basal medium or the proportional concentration of these components; 3) 200-6000 IU/mL of IL-2, 5-100 ng/mL of IL-7, 5-100 ng/mL of IL-15, 1-100 µg/mL of PD-1 antibodies, serum, dual antibiotics and basal medium or the proportional concentration of these components; 4) 200-6000 IU/mL of IL-2, 5-100 ng/mL of IL-7, 5-100 ng/mL of IL-15, 1-100 µg/mL of PD-1 antibodies, 1-100 µg/mL of GITR antibodies and 1-500 µM of TWS119, serum, dual antibiotics and basal medium or the proportional concentration of these components; 5) 200-6000 IU/mL of IL-2, 5-100 ng/mL of IL-7, 5-100 ng/mL of IL-15, 1-100 µg/mL of PD-1 antibodies, 1-100 µg/mL of TIGIT antibodies and 200-5000 U/mL of GM-CSF, serum, dual antibiotics and basal medium or the proportional concentration of these components; 6) 200-6000 IU/mL of IL-2, 5-100 ng/mL of IL-7, 5-100 ng/mL of IL-15, 1-100 µg/mL of PD-1 antibodies, 1-100 µg/mL of TIGIT antibodies, 1-100 µg/mL of CD40 antibodies, 1-100 µg/mL of OX-40 antibodies and 1×10⁸-5×10⁸/mL of autologous platelets, serum, dual antibiotics and basal medium or the proportional concentration of these components; 7) 200-6000 IU/mL of IL-2, 5-100 ng/mL of IL-7, 5-100 ng/mL of IL-15, 1-100 µg/mL of PD-1 antibodies, 1-100 µg/mL of CTLA-4 antibodies, 1-100 µg/mL of CD137 antibodies, 1-100 µg/mL of GITR antibodies, 200-5000 U/mL of GM-CSF and 1×10⁸-5×10⁸/mL of allogeneic platelets, serum, dual antibiotics and basal medium or the proportional concentration of these components,
m) the density of the first population of TIL cell in step (2) is 1.0×10⁵/mL~1.0× 10⁶/mL; preferably, 2.0×10⁵/mL~1.0×10⁶/mL; more preferably, 2.5×10⁵/mL~3.5× 10⁶/mL,
n) the contacting in step (2) lasts for 1-3 days; preferably, lasts for 2-3 days,
o) the TIL cell expansion medium described in step (3) includes any one of the following combinations of the components:
1) 200-500 IU/mL of IL-2, 5-50 ng/mL of IL-7, 5-50 ng/mL of IL-15, 1-50 µg/mL of PD-1 antibodies, 200-1000 U/mL GM-CSF, serum, dual antibiotics, and basal medium or the proportional concentration of these components; 2) 200-6000 IU/mL of IL-2, 5-100 ng/mL of IL-7, 5-100 ng/mL of IL-15, 1-100 µg/mL of PD-1 antibodies, 5-100 ng/mL of IL-21, 5-100 ng/mL of IL-12, 1-100 µg/mL of CD137 antibodies and 1-100 µg/mL of GITR antibodies, serum, dual antibiotics and basal medium or the proportional concentration of these components; 3) 200-6000 IU/mL IL-2, 5-100 ng/mL IL-7, 5-100 ng/mL IL-15, 1-100 µg/mL of PD-1 antibodies, serum, dual antibiotics and basal medium or the proportional concentration of these components; 4) 200-6000 IU/mL of IL-2, 5-100 ng/mL of IL-7, 5-100 ng/mL of IL-15, 1-100 µg/mL of PD-1 antibodies, 1-100 µg/mL of GITR antibodies and 1-500 µM of TWS119, serum, dual antibiotics and basal medium or the proportional concentration of these components; 5) 200-6000 of IU/mL IL-2, 5-100 ng/mL of IL-7, 5-100 ng/mL of IL-15, 1-100 µg/mL of PD-1 antibodies, 1-100 µg/mL of TIGIT antibodies and 200-5000 U/mL of GM-CSF, serum, dual antibiotics and basal medium or the proportional concentration of these components; 6) 200-6000 IU/mL of IL-2, 5-100 ng/mL of IL-7, 5-100 ng/mL of IL-15, 1-100 µg/mL of PD-1 antibodies, 1-100 µg/mL of TIGIT antibodies, 1-100 µg/mL of CD40 antibodies, 1-100 µg/mL of OX-40 antibodies and 1×10⁸-5×10⁸/mL of autologous platelets, serum, dual antibiotics and basal medium or the proportional concentration of these components; 7) 200-6000 IU/mL of IL-2, 5-100 ng/mL of IL-7, 5-100 ng/mL of IL-15, 1-100 µg/mL of PD-1 antibodies, 1-100 µg/mL of CTLA-4 antibodies, 1-100 µg/mL of CD137 antibodies, 1-100 µg/mL of GITR antibodies, 200-5000 U/mL of GM-CSF and 1×10⁸-5×10⁸/mL allogeneic platelets, serum, dual antibiotics and basal medium or the proportional concentration of these components,
p) the incubation of step (3) lasts at least 5 days,
q) the incubation of step (3) lasts at most 20 days,
r) the temperature of the incubation of step (3) is 30-42°C, preferably 37°C,
s) the concentration of CO2 in the incubation of step (3) is 5%,
t) the tumor cell-containing tissue is a tumor tissues or body fluids of subjects having a cancer selected from the group consisting of a gastric cancer, a thyroid tumor, a gallbladder cancer, a cholangiocarcinoma, a lung cancer, a melanoma, a head and neck cancer, a breast cancer, an ovarian cancer, a cervical cancer, a liver cancer, a colorectal cancer, a cerebral glioma, a pancreatic cancer, a bladder cancer, a prostate cancer, a renal cancer, an osteosarcoma,
preferably, the basal medium is any one selected from the group consisting of AIM-V, X-VIVO, DMEM, RPMI1640, OpTmizer^{™}, and FUJIFILM Irvin MHM-C,
preferably, the serum is selected from the group consisting of human AB serum, autologous serum or animal-derived serum, and preferably, the concentration of the serum is v/v1-10 %.

8. Use of the compound of Formula (I) or pharmaceutically acceptable salt, isomer, racemate, solvate, hydrate or prodrug thereof in the manufacture of a medicament for treating tumors 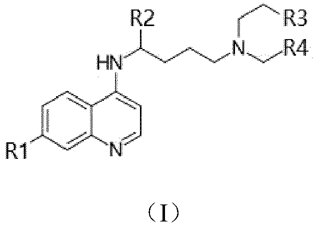
wherein, R1-R4 are each independently selected from the group consisting of H, alkyl, halogen, hydroxyl, amino, and the alkyl is optionally substituted by a group selected from the group consisting of H, C1-C6 alkyl, halogen, hydroxyl, amino, carboxyl,
preferably, R1-R4 are each independently selected from the group consisting of H, C1-C6 alkyl, halogen, hydroxyl, and said C1-C6 alkyl is optionally substituted by a group selected from the group consisting of H, C1-C6 alkyl, halogen, hydroxyl.

9. The use according to claim 8, wherein,
the number of immune cells contained in the tumor cell killing agent is at least 10⁸ cells, and/or
the tumor cell killing agent is an immune cell having anti-tumor activity, which is in the form of a cell cryopreservant preparation, and/or
the cell density of the cell cryopreservant preparation is 1.0×10⁸ cells /mL~2.0× 10⁸ cells /mL, and/or
the immune cells with anti-tumor activity are one or more selected from the group consisting of: a LAK, a DC, a CIK, a DC-CIK, a CAR-T, a TCR-T, a NK, a CAR- NK, a TIL.

10. The use according to claim 9, wherein, the immune cells having anti-tumor activity are TILs, preferably, the TILs are produced by the method described in claim 6 or 7.

11. The use according to claim 8, wherein, the tumor is one or more selected from the group consisting of squamous cell carcinoma, basal cell carcinoma, adenoma, adenocarcinoma, papillary adenoma, papillary adenocarcinoma, cystadenoma, pancreatic carcinoma, cystadenocarcinoma, pleomorphic adenoma, malignant pleomorphic adenoma, papilloma, transitional epithelium cancer, fibroma, fibrosarcoma, fibrous histiocytoma, malignant fibrous histiocytoma, lipoma, liposarcoma, leiomyoma, leiomyosarcoma, rhabdomyoma, rhabdomyosarcoma, hemangioma, angiosarcoma, lymphangioma, lymphangiosarcoma, osteoma, osteosarcoma, chondroma, chondrosarcoma, synovial tumor, synovial sarcoma, lymphoma, leukemia, neurofibroma, neurofibrosarcoma, schwannoma, malignant schwannoma, glioblastoma, malignant glioma, medulloblastoma, meningioma, malignant meningioma, ganglioneuroma, neuroblastoma, pigmented nevus, melanoma, molar pregnancy, chorionic epithelium carcinoma, seminoma, dysgerminoma, embryonal carcinoma, teratoma, malignant teratoma, nasal cavity carcinoma, nasopharyngeal carcinoma, sinus carcinoma, Burkitt lymphoma, pituitary tumor, lip carcinoma, multiple myeloma /plasmacytoma, gallbladder cancer, cholangiocarcinoma, lung cancer, non-small cell lung cancer, non-Hodgkin's lymphoma, peritoneal cancer, liver cancer, cervical cancer, anal cancer, testicular cancer, myelodysplasia, bone cancer, laryngeal cancer, Hodgkin lymphoma, Waldenstrom macroglobulinemia, colorectal cancer, thyroid cancer, parathyroid cancer, mesothelioma, malignant mesothelioma, acute lymphoblastic leukemia, acute myeloid leukemia, giant lymph node hyperplasia, basal cell tumor, oral cavity cancer, oropharyngeal cancer, Kaposi's sarcoma, ovarian cancer, Langerhans cell histiocytosis, appendix cancer, dermatofibrosarcoma protuberans, chronic lymphocytic leukemia, chronic myeloid leukemia, Merkel cells cancer, brain tumor, urethral cancer, male breast cancer, bladder cancer, skin cancer, cutaneous T-cell lymphoma, prostate cancer, breast cancer, gestational trophoblastic disease, soft tissue sarcoma, ovarian germ cell tumor, renal cancer, esophageal cancer, Pheochromocytoma/paraganglioma, Sézary syndrome, fallopian tube cancer, head and neck cancer, salivary gland, Ewing sarcoma, gastric cancer, gastrointestinal carcinoid, vulvar cancer, gastrointestinal stromal tumor, small bowel cancer, extragonadal germ cell tumor, thymoma, hypopharyngeal carcinoma, small cell lung cancer, mycosis fungoides, islet cell tumor, intraocular melanoma, vaginal carcinoma, penile carcinoma, occult primary squamous neck carcinoma, primary central nervous system lymphoma, uterine cancer, endometrial cancer, uterine sarcoma.

12. A method for treating solid tumors, comprising administering the pharmaceutical composition according to any one of claims 1-7 to a patient having a solid tumor.

13. The method according to claim 12, wherein, the pharmaceutical composition comprises autologous tumor infiltrating lymphocytes (TIL) of the patient having a solid tumor and the compound of Formula (I);
preferably, the compound of Formula (I) is hydroxychloroquine,
preferably, the method includes (1) first administering the compound of Formula (I) to the solid tumor patient; (2) infusing the autologous TILs to the solid tumor patient.

14. The method according to claim 13, wherein, the hydroxychloroquine is administered at a dose of 400mg/day-800mg/day, and/or,
the autologous TILs are prepared by the method described in claim 6 or 7, and/or
the hydroxychloroquine is administered on any 1 day, 2 days or 3 days of the time period lasting from the 6th day to the 3rd day before the infusion of the autologous TILs, and/or
the autologous TILs are in the form of a cell cryopreservant preparation, and/or
the autologous TILs are infused through intravenous infusion.

15. The method according to claim 14, wherein the cell cryopreservant preparation comprises a cryopreservant, and/or
the number of said autologous TILs infused is 1.0×10¹⁰ cells -1.2×10¹¹ cells, and/or
the cell density of the cell cryopreservant preparation is 1.0×10⁸ cells /mL~2.0× 10⁸ cells /mL, and/or
the volume of the cell cryopreservant preparation infused is 200mL-600mL.

16. The method according to claim 12, wherein, the solid tumor comprises: squamous cell carcinoma, basal cell carcinoma, adenoma, adenocarcinoma, papillary adenoma, papillary adenocarcinoma, cystadenoma, pancreatic carcinoma, cystadenocarcinoma, pleomorphic adenoma, malignant pleomorphic adenoma, papilloma, transitional epithelium cancer, fibroma, fibrosarcoma, fibrous histiocytoma, malignant fibrous histiocytoma, lipoma, liposarcoma, leiomyoma, leiomyosarcoma, rhabdomyoma, rhabdomyosarcoma, hemangioma, angiosarcoma, lymphangioma, lymphangiosarcoma, osteoma, osteosarcoma, chondroma, chondrosarcoma, synovial tumor, synovial sarcoma, lymphoma, leukemia, neurofibroma, neurofibrosarcoma, schwannoma, malignant schwannoma, glioblastoma, malignant glioma, medulloblastoma, meningioma, malignant meningioma, ganglioneuroma, neuroblastoma, pigmented nevus, melanoma, molar pregnancy, chorionic epithelium carcinoma, seminoma, dysgerminoma, embryonal carcinoma, teratoma, malignant teratoma, nasal cavity carcinoma, nasopharyngeal carcinoma, sinus carcinoma, Burkitt lymphoma, pituitary tumor, lip carcinoma, multiple myeloma /plasmacytoma, gallbladder cancer, cholangiocarcinoma, lung cancer, non-small cell lung cancer, non-Hodgkin's lymphoma, peritoneal cancer, liver cancer, cervical cancer, anal cancer, testicular cancer, myelodysplasia, bone cancer, laryngeal cancer, Hodgkin lymphoma, Waldenstrom macroglobulinemia, colorectal cancer, thyroid cancer, parathyroid cancer, mesothelioma, malignant mesothelioma, acute lymphoblastic leukemia, acute myeloid leukemia, giant lymph node hyperplasia, basal cell tumor, oral cavity cancer, oropharyngeal cancer, Kaposi's sarcoma, ovarian cancer, Langerhans cell histiocytosis, appendix cancer, dermatofibrosarcoma protuberans, chronic lymphocytic leukemia, chronic myeloid leukemia, Merkel cells cancer, brain tumor, urethral cancer, male breast cancer, bladder cancer, skin cancer, cutaneous T-cell lymphoma, prostate cancer, breast cancer, gestational trophoblastic disease, soft tissue sarcoma, ovarian germ cell tumor, renal cancer, esophageal cancer, Pheochromocytoma/paraganglioma, Sézary syndrome, fallopian tube cancer, head and neck cancer, salivary gland, Ewing sarcoma, gastric cancer, gastrointestinal carcinoid, vulvar cancer, gastrointestinal stromal tumor, small bowel cancer, extragonadal germ cell tumor, thymoma, hypopharyngeal carcinoma, small cell lung cancer, mycosis fungoides, islet cell tumor, intraocular melanoma, vaginal carcinoma, penile carcinoma, occult primary squamous neck carcinoma, primary central nervous system lymphoma, uterine cancer, endometrial cancer, uterine sarcoma.

17. The method according to claim 12, wherein, the method further comprises efficacy evaluation.

18. The method according to claim 17, wherein, the efficacy evaluation comprises tumor imaging evaluation and/or tumor marker expression level evaluation.
